(19) Europäisches Patentamt
European Patent Office
Office européen des brevets

(11) EP 1 363 668 B1

(12) EUROPEAN PATENT SPECIFICATION

(45) Date of publication and mention of the grant of the patent:
**15.08.2007 Bulletin 2007/33**

(21) Application number: **02707556.3**

(22) Date of filing: **25.01.2002**

(51) Int Cl.:
***A61K 31/397*** *(2006.01)* ***A61K 31/765*** *(2006.01)*
***A61P 9/00*** *(2006.01)*

(86) International application number:
**PCT/US2002/002010**

(87) International publication number:
**WO 2002/058733 (01.08.2002 Gazette 2002/31)**

# (54) COMBINATIONS OF BILE ACID SEQUESTRANT(S) AND STEROL ABSORPTION INHIBITOR(S) AND TREATMENTS FOR VASCULAR INDICATIONS

KOMBINATIONEN VON GALLENSÄURESEQUESTRIERMITTELN UND HEMMERN DER STEROLABSORPTION ZUR BEHANDLUNG VON KARDIOVASKULÄREN INDIKATIONEN

COMBINAISONS DE CHELATEUR(S) DES ACIDES BILIAIRES ET D'INHIBITEUR(S) D'ABSORPTION DES STEROLS, ET TRAITEMENTS POUR TROUBLES VASCULAIRES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **26.01.2001 US 264600 P**
**21.09.2001 US 323842 P**

(43) Date of publication of application:
**26.11.2003 Bulletin 2003/48**

(60) Divisional application:
**07003151.3 / 1 785 144**

(73) Proprietor: **SCHERING CORPORATION**
**Kenilworth, NJ 07033-0530 (US)**

(72) Inventors:
- **DAVIS, Harry, R.**
**Berkeley Heights, NJ 07922 (US)**
- **KOSOGLOU, Teddy**
**Jamison, Bucks County, PA 18929-1178 (US)**

(74) Representative: **Klusmann, Peter**
**Hoffmann - Eitle**
**Patent- und Rechtsanwälte**
**Arabellastrasse 4**
**81925 München (DE)**

(56) References cited:
**WO-A-00/38725** **WO-A-02/26729**
**WO-A-02/50027** **WO-A-02/50060**
**WO-A-02/50068** **WO-A-95/35277**
**WO-A-02/064094** **WO-A-02/081454**

- **THOMPSON G. R. ET AL: "Novel lipid regulating drugs" EXPERT OPINION ON INVESTIGATIONAL DRUGS, vol. 9, no. 11, December 2000 (2000-12), XP008011782**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to compositions and therapeutic combinations comprising bile acid sequestrants and sterol absorption inhibitors of formula (II) for treating hyperlipidemic conditions associated with atherosclerosis, hypercholesterolemia and other vascular conditions in mammals.

### BACKGROUND OF THE INVENTION

Atherosclerotic coronary heart disease (CHD) represents the major cause for death and vascular morbidity in the western world. Risk factors for atherosclerotic coronary heart disease include hypertension, diabetes mellitus, family history, male gender, cigarette smoke and serum cholesterol. A total cholesterol level in excess of 225-250 mg/dl is associated with significant elevation of risk of CHD.

Cholesteryl esters are a major component of atherosclerotic lesions and the major storage form of cholesterol in arterial wall cells. Formation of cholesteryl esters is also a key step in the intestinal absorption of dietary cholesterol. Thus, inhibition of cholesteryl ester formation and reduction of serum cholesterol can inhibit the progression of atherosclerotic lesion formation, decrease the accumulation of cholesteryl esters in the arterial wall, and block the intestinal absorption of dietary cholesterol.

The regulation of whole-body cholesterol homeostasis in mammals and animals involves the regulation of dietary cholesterol and modulation of cholesterol biosynthesis, bile acid biosynthesis and the catabolism of the cholesterol-containing plasma lipoproteins. The liver is the major organ responsible for cholesterol biosynthesis and catabolism and, for this reason, it is a prime determinant of plasma cholesterol levels. The liver is the site of synthesis and secretion of very low density lipoproteins (VLDL) which are subsequently metabolized to low density lipoproteins (LDL) in the circulation. LDL are the predominant cholesterol-carrying lipoproteins in the plasma and an increase in their concentration is correlated with increased atherosclerosis. When intestinal cholesterol absorption is reduced, by whatever means, less cholesterol is delivered to the liver. The consequence of this action is decreased hepatic lipoprotein (VLDL) production and an increase in the hepatic clearance of plasma cholesterol, mostly as LDL. Thus, the net effect of inhibiting intestinal cholesterol absorption is a decrease in plasma cholesterol levels.

Bile acid sequestrants, such as cholestyramine and colestipol, can lower intrahepatic cholesterol and promote the synthesis of apo B/E (LDL) receptors which bind LDL from plasma to further reduce cholesterol levels in the blood.

U.S. Patents Nos. 5,767,115, 5,624,920, 5,668,990, 5,656,624 and 5,688,787, respectively, disclose hydroxy-substituted azetidinone compounds and substituted β-lactam compounds useful for lowering cholesterol and/or in inhibiting the formation of cholesterol-containing lesions in mammalian arterial walls. U.S. Patents Nos. 5,846,966 and 5,661,145, respectively, disclose hydroxy-substituted azetidinone compounds or substituted β-lactam compounds in combination with HMG CoA reductase inhibitors for preventing or treating atherosclerosis and reducing plasma cholesterol levels.

PCT Patent Application No. WO 00/38725 discloses cardiovascular therapeutic combinations including an ileal bile acid transport inhibitor or cholesteryl ester transport protein inhibitor in combination with a fibric acid derivative, nicotinic acid derivative, microsomal triglyceride transfer protein inhibitor, cholesterol absorption antagonist, phytosterol, stanol, antihypertensive agent or bile acid sequestrant.

U.S. Patent No. 5,698,527 discloses ergostanone derivatives substituted with disaccharides as cholesterol absorption inhibitors, employed alone or in combination with certain other cholesterol lowering agents, which are useful in the treatment of hypercholesterolemia and related disorders.

Despite recent improvements in the treatment of vascular disease, there remains a need in the art for improved compositions and treatments for hyperlipidaemia, atherosclerosis and other vascular conditions.

WO 9535277 relates to the use of substituted azetidinone derivatives. No combination with bile acid sequestrants is mentioned in this document.

Thompson G.R. et al: "Expert opinion on investigational drugs", Vol. 9, No. 11, December 2000 describes combination therapies. The specific combination of compounds of the invention is not mentioned in this document.

WO 02 081454 A relates to b-aryl-a-oxysubstituted alkylcarboxylic acids of the general formula (I), their derivatives, their analogs, their tautomeric forms, their stereoisomers, their polymorphs, their pharmaceutically acceptable salts, their pharmaceutically acceptable solvates and pharmaceutically acceptable compositions containing them. Combinations of such a compound with various other compounds are also briefly mentioned.

WO 02 50068 A and WO 02 50060 A describe diphenyl azetidinones, a method for the production thereof, medicaments containing these compounds, and their use. The compounds of these documents are said to be suited for use e.g. as hypolipidemic drugs. Combinations with various other compounds are also briefly mentioned.

WO 02 50027 A claims a priority earlier than the priority date of the present application and represents, as far

as entitled to the claimed priority, prior art under Art. 54(3) EPC. It relates to 1,2 Diphenylazetidinones, a method for producing the same, medicaments containing them, and their use for treating disorders of the lipid metabolism. This document also briefly mentions combinations of the compounds claimed therein with various other biologically active compounds.

## SUMMARY OF THE INVENTION

In one embodiment, the present invention provides a composition as defined in claim 1 and the claims dependent thereon.

In addition, therapeutic combinations are provided as defined in claim 2 and its dependent claims.

Pharmaceutical compositions for the treatment or prevention of a vascular condition selected from hyperlipidaemia, atherosclerosis, hypercholesterolemia, diabetes, obesity comprising a therapeutically effective amount of the above compositions or therapeutic combinations and a pharmaceutically acceptable carrier also are provided. Uses for manufacturing a medicament for treating or preventing a vascular condition, selected from hyperlipidaemia, atherosclerosis, hypercholesterolemia diabetes, obesity also are provided.

## DETAILED DESCRIPTION

Bile acid sequestrants (insoluble anion exchange resins) bind bile acids in the intestine, interrupting the enterohepatic circulation of bile acids and causing an increase in the faecal excretion of steroids. Use of bile acid sequestrants is desirable because of their non-systemic mode of action. Bile acid sequestrants can lower intrahepatic cholesterol and promote the synthesis of apo B/E (LDL) receptors which bind LDL from plasma to further reduce cholesterol levels in the blood.

Suitable bile acid sequestrants are cholestyramine (a styrene-divinylbenzene copolymer containing quaternary ammonium cationic groups capable of binding bile acids, such as QUESTRAN® or QUESTRAN LIGHT® which are available from Bristol-Myers Squibb), colestipol (a copolymer of diethylenetriamine and 1-chloro-2,3-epoxypropane, such as COLESTID® tablets which are available from Pharmacia), colesevelam hydrochloride (such as WelChol® Tablets (poly(allylamine hydrochloride) cross-linked with epichlorohydrin and alkylated with 1-bromodecane and (6-bromohexyl)-trimethylammonium bromide) which are available from Sankyo), and mixtures thereof.

The bile acid sequestrant(s) as defined above are administered in a therapeutically effective amount to treat the specified condition, for example in a daily dose preferably ranging from 1 to 50 grams per day, and more preferably 2 to 16 grams per day, given in a single dose or 2-4 divided doses. The exact dose, however, is determined by the attending clinician and is dependent on such factors as the potency of the compound administered, the age, weight, condition and response of the patient.

The phrase "therapeutically effective amount" means that amount of a therapeutic agent of the composition, such as the bile acid sequestrant(s) as defined above, sterol absorption inhibitory of formula (II) and pharmacological or therapeutic agents described below, that will elicit a biological or medical response of a tissue, system, animal or mammal that is being sought by the administrator (such as a researcher, doctor or veterinarian) which includes alleviation of the symptoms of the condition or disease being treated and the prevention, slowing or halting of progression of one or more conditions, for example vascular conditions, such as hyperlipidemia (for example atherosclerosis, hypercholesterolemia or sitosterolemia), diabetes, obesity

As used herein, "combination therapy" or "therapeutic combination" means the administration of two or more therapeutic agents, such as bile acid sequestrant(s) and sterol absorption inhibitor(s), to prevent or treat a condition, for example a vascular condition, such as hyperlipidaemia (for example atherosclerosis, hypercholesterolemia or sitosterolemia), diabetes, obesity. As used herein, "vascular" comprises cardiovascular, cerebrovascular and combinations thereof. Such administration includes coadministration of these therapeutic agents in a substantially simultaneous manner, such as in a single tablet or capsule having a fixed ratio of active ingredients or in multiple, separate capsules for each therapeutic agent. Also, such administration includes use of each type of therapeutic agent in a sequential manner. In either case, the treatment using the combination therapy will provide beneficial effects in treating the condition. A potential advantage of the combination therapy disclosed herein may be a reduction in the required amount of an individual therapeutic compound or the overall total amount of therapeutic compounds that are effective in treating the condition. By using a combination of therapeutic agents, the side effects of the individual compounds can be reduced as compared to a monotherapy, which can improve patient compliance. Also, therapeutic agents can be selected to provide a broader range of complementary effects or complimentary modes of action.

As discussed above, the compositions, pharmaceutical compositions and therapeutic combinations of the present invention comprise one or more substituted azetidinone sterol absorption inhibitors according to formula (II) discussed in detail below. As used herein, "sterol absorption inhibitor" means a compound capable of inhibiting the absorption of one or more sterols, when administered in a therapeutically effective (sterol absorption inhibiting) amount

to a mammal.

Sterol absorption inhibitors useful in the compositions, therapeutic combinations and methods of the present invention are represented by Formula (II) which will be shown later.

Compounds of the invention have at least one asymmetrical carbon atom and therefore all isomers, including enantiomers, stereoisomers, rotamers, tautomers and racemates of the compounds of Formula (II) (where they exist) are contemplated as being part of this invention. The invention includes d and l isomers in both pure form and in admixture, including racemic mixtures. Isomers can be prepared using conventional techniques, either by reacting optically pure or optically enriched starting materials or by separating isomers of a compound of the Formula II.

Those skilled in the art will appreciate that for some of the compounds of the Formula II, one isomer will show greater pharmacological activity than other isomers.

Compounds of the invention with an amino group can form pharmaceutically acceptable salts with organic and inorganic acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salt is prepared by contacting the free base form with a sufficient amount of the desired acid to produce a salt. The free base form may be regenerated by treating the salt with a suitable dilute aqueous base solution such as dilute aqueous sodium bicarbonate. The free base form differs from its respective salt form somewhat in certain physical properties, such as solubility in polar solvents, but the salt is otherwise equivalent to its respective free base forms for purposes of the invention.

Certain compounds of the invention are acidic (e.g., those compounds which possess a carboxyl group). These compounds form pharmaceutically acceptable salts with inorganic and organic bases. Examples of such salts are the sodium, potassium, calcium, aluminum, gold and silver salts. Also included are salts formed with pharmaceutically acceptable amines such as ammonia, alkyl amines, hydroxyalkylamines, N-methylglucamine.

As used herein, "solvate" means a molecular or ionic complex of molecules or ions of solvent with those of solute (for example, one or more compounds of Formula II, isomers of the compounds of Formula II. Examples of useful solvents include polar, protic solvents such as water and/or alcohols (for example methanol).

In the present invention, a sterol inhibitor of Formula (II) (ezetimibe) useful in the compositions, therapeutic combinations and methods of the present invention is represented by Formula (II) below:

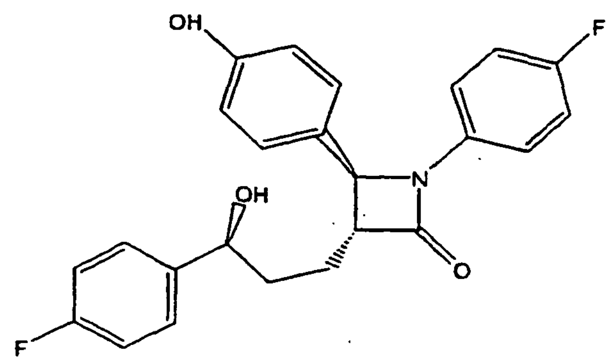

(II)

or pharmaceutically acceptable salts or solvates of the compound of Formula (II).

Compounds of Formula II can be prepared by a variety of methods well know to those skilled in the art, for example such as are disclosed in U.S. Patents Nos. 5,631,365, 5,767,115, 5,846,966, 6,207,822 and U.S. Provisional Patent Application No. 60/279,288 filed March 28, 2001, and PCT Patent Application WO 93/02048, and in the Example below. For example, suitable compounds of Formula II can be prepared by a method comprising the steps of:

(a) treating with a strong base a lactone of the Formula A or B:

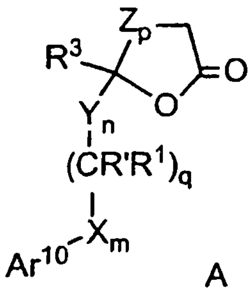

A

or

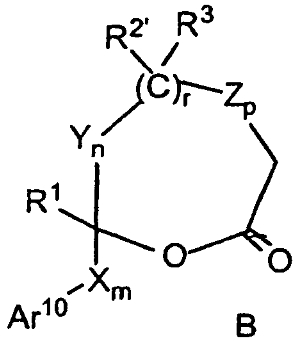

B

wherein R' and $R^{2'}$ are R and $R^2$, respectively, or are suitably protected hydroxy groups; $Ar^{10}$ is $Ar^1$, a suitably protected hydroxy-substituted aryl or a suitably protected amino-substituted aryl; and the remaining variables are as defined above for Formula I, provided that in lactone of formula B, when n and r are each zero, p is 1-4;

(b) reacting the product of step (a) with an imine of the formula

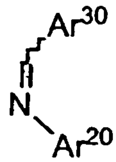

wherein $Ar^{2O}$ is $Ar^2$, a suitably protected hydroxy-substituted aryl or a suitably protected amino-substituted aryl; and $Ar^{30}$ is $Ar^3$, a suitably protected hydroxy-substituted aryl or a suitably protected amino-substituted aryl;

c) quenching the reaction with an acid;

d) optionally removing the protecting groups from R', $R^{2'}$, $Ar^{10}$, $Ar^{2O}$ and $Ar^{30}$, when present; and

e) optionally functionalizing hydroxy or amino substituents at R, $R^2$, $Ar^1$, $Ar^2$ and $Ar^3$.

Using the lactones shown above, compounds of Formula IA and IB are obtained as follows:

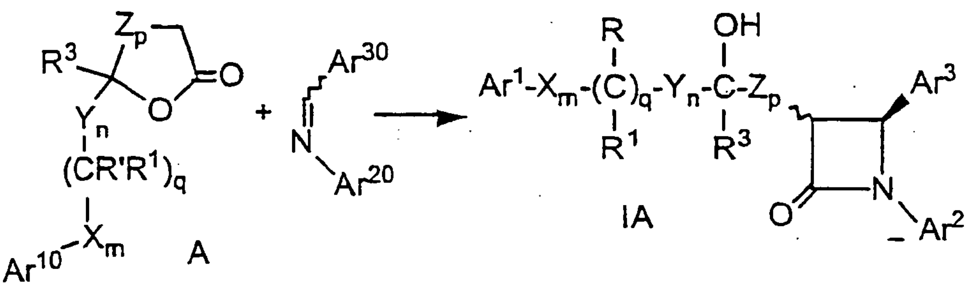

A

IA

wherein the variables are as defined above; and

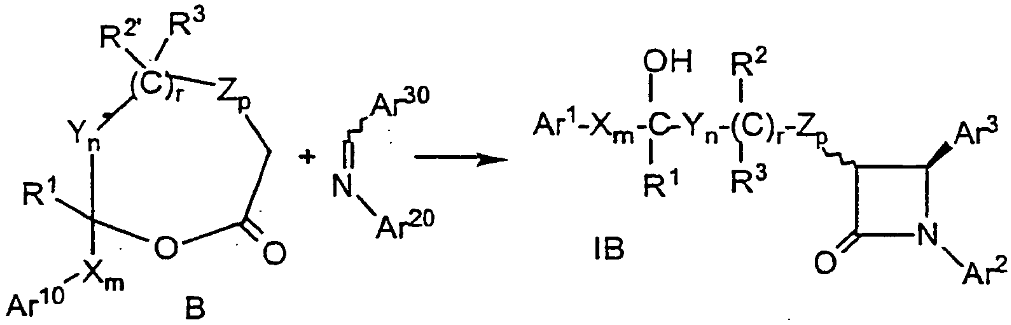

wherein the variables are as defined above.

The daily dose of the sterol absorption inhibitor of formula (II) preferably ranges from 0.1 to 1000 mg per day, and more preferably 0.25 to 50 mg/day, given in a single dose or 2-4 divided doses. The exact dose, however, is determined by the attending clinician and is dependent on the potency of the compound administered, the age, weight, condition and response of the patient.

For administration of pharmaceutically acceptable salts of the above compounds, the weights indicated above refer to the weight of the acid equivalent or the base equivalent of the therapeutic compound derived from the salt.

In one embodiment, the compositions or therapeutic combinations can further comprise one or more pharmacological or therapeutic agents or drugs such as cholesterol biosynthesis inhibitors and/or lipid-lowering agents discussed below.

In another embodiment, the composition can further comprise one or more cholesterol biosynthesis inhibitors coadministered with or in combination with the bile acid sequestrant(s) and sterol absorption inhibitor(s) discussed above.

Examples of cholesterol biosynthesis inhibitors for use in the compositions, therapeutic combinations and methods of the present invention include competitive inhibitors of HMG CoA reductase, the rate-limiting step in cholesterol biosynthesis, squalene synthase inhibitors, squalene epoxidase inhibitors and mixtures thereof. Examples of suitable HMG CoA reductase inhibitors include statins such as lovastatin (for example MEVACOR® which is available from Merck & Co.), pravastatin (for example PRAVACHOL® which is available from Bristol Meyers Squibb), fluvastatin, simvastatin (for example ZOCOR® which is available from Merck & Co.), atorvastatin, cerivastatin, CI-981 and pitavastatin (such as NK-104 of Negma Kowa of Japan); HMG CoA synthetase inhibitors, for example L-659,699 ((E,E)-11-[3'R-(hydroxy-methyl)-4'-oxo-2'R-oxetanyl]-3,5,7R-trimethyl-2,4-undecadienoic acid); squalene synthesis inhibitors, for example squalestatin 1; and squalene epoxidase inhibitors, for example, NB-598 ((E)-N-ethyt-N-(6,6-dimethyl)-2-hepten-4-ynyl)-3-[(3,3'-bithiophen-5-yl)methoxy]benzene-methanamine hydrochloride) and other sterol biosynthesis inhibitors such as DMP-565. Preferred HMG CoA reductase inhibitors include lovastatin, pravastatin and simvastatin. The most preferred HMG CoA reductase inhibitor is simvastatin.

Generally, a total daily dosage of cholesterol biosynthesis inhibitor of formula (II) can range from 0.1 to 160 mg per day, and preferably 0.2 to 80 mg/day in single or 2-3 divided doses.

In another preferred embodiment, the composition comprises the compound of Formula (II) in combination with one or more bile acid sequestrants and one or more cholesterol biosynthesis inhibitors. In this embodiment, preferably the bile acid sequestrant is selected from cholestyramine and/or colestipol. Preferably the cholesterol biosynthesis inhibitor comprises one or more HMG CoA reductase inhibitors, such as, for example, lovastatin, pravastatin and/or simvastatin. More preferably, the composition or treatment comprises the compound of Formula (II) in combination with simvastatin and cholestyramine or colestipol.

Also useful with the present invention are compositions or therapeutic combinations that can further comprise at least one (one or more) activators for peroxisome proliferator-activated receptors (PPAR). The activators act as agonists for the peroxisome proliferator-activated receptors. Three subtypes of PPAR have been identified, and these are designated as peroxisome proliferator-activated receptor alpha (PPARα), peroxisome proliferator-activated receptor gamma (PPARγ) and peroxisome proliferator-activated receptor delta (PPARδ). It should be noted that PPARδ is also referred to in the literature as PPARβ and as NUC1, and each of these names refers to the same receptor.

PPARα regulates the metabolism of lipids. PPARα is activated by fibrates and a number of medium and long-chain fatty acids, and it is involved in stimulating β-oxidation of fatty acids. The PPARγ receptor subtypes are involved in activating the program of adipocyte differentiation and are not involved in stimulating peroxisome proliferation in the liver. PPARδ has been identified as being useful in increasing high density lipoprotein (HDL) levels in humans. See, e.g., WO 97/28149.

PPARα activator compounds are useful for, among other things, lowering triglycerides, moderately lowering LDL levels and increasing HDL levels. Useful examples of PPARα activators include fibrates.

Examples of suitable fibric acid derivatives ("fibrates") include clofibrate (such as ethyl 2-(p-chlorophenoxy)-2-methyl-propionate, for example ATROMID-S® Capsules which are commercially available from Wyeth-Ayerst); gemfibrozil (such as 5-(2,5-dimethylphenoxy)-2,2-dimethylpentanoic acid, for example LOPID® tablets which are commercially available from Parke Davis); ciprofibrate (C.A.S. Registry No. 52214-84-3, see U.S. Patent No. 3,948,973 ); bezafibrate (C.A.S. Registry No. 41859-67-0, see U.S. Patent No. 3,781,328); clinofibrate (C.A.S. Registry No. 30299-08-2, see U.S. Patent No. 3,716,583 ); binifibrate (C.A.S. Registry No. 69047-39-8, see BE 884722 ); lifibrol (C.A.S. Registry No. 96609-16-4); fenofibrate (such as TRICOR® micronized fenofibrate (2-[4-(4-chlorobenzoyl) phenoxy]-2-methyl-propanoic acid, 1-methylethyl ester) which is commercially available from Abbott Laboratories or LIPANTHYL® micronized fenofibrate which is commercially available from Labortoire Founier, France) and mixtures thereof. These compounds can be used in a variety of forms, including acid form, salt form, racemates, enantiomers, zwitterions and tautomers

Other examples of PPARα activators useful with the practice of the present invention include suitable fluorophenyl compounds as disclosed in U.S. No. 6,028,109 certain substituted phenylpropionic compounds as disclosed in WO 00/75103 and PPARα activator compounds as disclosed in WO 98/43081.

Examples of suitable PPARγ activators include derivatives of glitazones or thiazolidinediones, such as, troglitazone (such as REZULIN® troglitazone (-5-[[4-[3,4-dihydro-6-hydroxy-2,5,7,8-tetramethyl-2H-1-benzopyran-2-yl)methoxy]phenyl] methyl]-2,4-thiazolidinedione) commercially available from Parke-Davis); rosiglitazone (such as AVANDIA® rosiglitazone maleate (-5-[[4-[2-(methyl-2-pyridinylamino)ethoxy] phenyl] methyl]-2,4-thiazolidinedione, (Z) -2-butenedioate) (1:1) commercially available from SmithKline Beecham) and pioglitazone (such as ACTOS™ pioglitazone hydrochloride (5-[[4-[2-(5-ethyl-2-pyridinyl)ethoxy]phenyl)methyl]-2;4-]thiazolidinedione monohydrochloride) commercially available from Takeda Pharmaceuticals). Other useful thiazolidinediones include ciglitazone, englitazone, darglitazone and BRL 49653 as disclosed in WO 98/05331 PPARγ activator compounds disclosed in WO 00/76488; and PPARγ activator compounds disclosed in U.S. Patent No. 5,994,554.

Other useful PPARγ activator compounds include certain acetylphenols as disclosed in U.S. Patent No. 5,859,051 certain quinoline phenyl compounds as disclosed in WO 99/20275; aryl compounds as disclosed by WO 99/38845; certain 1,4-disubstituted phenyl compounds as disclosed in WO 00/63161; certain aryl compounds as disclosed in WO 01/00579 ; benzoic acid compounds as disclosed in WO 01/12612 and WO 01/12187; and substituted 4-hydroxyphenylalconic acid compounds as disclosed in WO 97/31907.

PPARδ compounds are useful for, among other things; lowering triglyceride levels or raising HDL levels. Examples of PPARδ activators include suitable thiazole and oxazole derivates, such as C.A.S. Registry No. 317318-32-4, as disclosed in WO 01/00603; certain fluoro, chloro or thio phenoxy phenylacetic acids as disclosed in WO 97/28149; suitable non-β-oxidizable fatty acid analogues as disclosed in U.S. Patent No. 5,093,365 and PPARδ compounds as disclosed in WO 99/04815.

Moreover, compounds that have multiple functionality for activating various combinations of PPARα, PPARγ and PPARδ are also useful with the practice of the present invention. Examples include certain substituted aryl compounds as disclosed in U.S. Patent No. 6,248,781; WO 00/23416; WO 00/23415; WO 00/23425; WO 00/23445; WO 00/23451; and WO 00/63153, are described as being useful PPARα and/or PPARγ activator compounds. Other examples of useful PPARα and/or PPARγ activator compounds include activator compounds as disclosed in WO 97/25042; activator compounds as disclosed in WO 00/63190; activator compounds as disclosed in WO 01/21181; biaryl-oxa(thia)zole compounds as disclosed in WO 01/16120; compounds as disclosed in WO 00/63196 and WO 00/63209; substituted 5-aryl-2,4-thiazolidinediones compounds as disclosed in U.S. Patent No. 6,008,237; arylthiazolidinedione and aryloxazolidinedione compounds as disclosed in WO 00/78312 and WO 00/78313G; GW2331 or (2-(4-[difluorophenyl]-1heptylureido) ethyl]phenoxy)-2-methylbutyric compounds as disclosed in WO 98/05331; aryl compounds as disclosed in U.S. Patent No. 6,166,049; oxazole compounds as disclosed in WO 01/17994; and dithiolane compounds as disclosed in WO 01/25225 and WO 01/25226.

Other useful PPAR activator compounds include substituted benzylthiazolidine-2,4-dione compounds as disclosed in WO 01/14349, WO 01/14350 and WO/01/04351; mercaptocarboxylic compounds as disclosed in WO 00/50392; ascofuranone compounds as disclosed in WO 00/53563; carboxylic compounds as disclosed in WO 99/46232; compounds as disclosed in WO 99/12534; benzene compounds as disclosed in WO 99/15520; o-anisamide compounds as disclosed in WO 01/21578; and PPAR activator compounds as disclosed in WO 01/40192.

The peroxisome proliferator-activated receptor(s) activator(s) are administered in a therapeutically effective amount to treat the specified condition, for example in a daily dose preferably ranging from 50 to 3000 mg per day, and more preferably 50 to 2000 mg per day, given in a single dose or 2-4 divided doses. The exact dose, however, is determined by the attending clinician and is dependent on such factors as the potency of the compound administered, the age, weight, condition and response of the patient.

In an alternative embodiment, the compositions of the present invention can further comprise one or more ileal bile acid transport ("IBAT") inhibitors (or apical sodium co-dependent bile acid transport ("ASBT") inhibitors) coadministered with or in combination with the bile acid sequestrant(s) and sterol absorption inhibitor of formula (II) discussed above. The IBAT inhibitors can inhibit bile acid transport to reduce LDL cholesterol levels. Examples of suitable IBAT inhibitors include benzothiepines such as therapeutic compounds comprising a 2,3,4,5-tetrahydro-1-benzothiepine 1,1-dioxide structure such as are disclosed in PCT Patent Application WO 00/38727.

Generally, a total daily dosage of IBAT inhibitor(s) can range from 0.01 to 1000 mg/day, and preferably 0.1 to 50 mg/day in single or 2-4 divided doses.

In another alternative embodiment, the compositions of the present invention can further comprise nicotinic acid (niacin) and/or derivatives thereof coadministered with or in combination with the bile acid sequestrant(s) and sterol absorption inhibitor of formula (II) discussed above.

As used herein, "nicotinic acid derivative" means a compound comprising a pyridine-3-carboxylate structure or a pyrazine-2-carboxylate structure, including acid forms, salts, esters, zwitterions and tautomers, where available. Examples of nicotinic acid derivatives include niceritrol, nicofuranose and acipimox (5-methyl pyrazine-2-carboxylic acid 4-oxide). Nicotinic acid and its derivatives inhibit hepatic production of VLDL and its metabolite LDL and increases HDL and apo A-1 levels. An example of a suitable nicotinic acid product is NIASPAN® (niacin extended-release tablets) which are available from Kos.

Generally, a total daily dosage of nicotinic acid or a derivative thereof can range from 500 to 10,000 mg/day, preferably 1000 to 8000 mg/day, and more preferably 3000 to 6000 mg/day in single or divided doses.

In another alternative embodiment, the compositions of the present invention can further comprise one or more AcylCoA:Cholesterol O-acyltransferase ("ACAT") Inhibitors, which can reduce LDL and HDL levels, coadministered with or in combination with the bile acid sequestrant(s) and sterol absorption inhibitor of formula (II) discussed above. ACAT is an enzyme responsible for esterifying excess intracellular cholesterol and may reduce the synthesis of VLDL, which is a product of cholesterol esterification, and overproduction of apo B-100-containing lipoproteins.

Examples of useful ACAT inhibitors include avasimibe ([[2,4,6-tris(1-methylethyl)phenyl]acetyl]sulfamic acid, 2,6-bis(1-methylethyl)phenyl ester, formerly known as CI-1011), HL-004, lecimibide (DuP-128) and CL-277082 (N-(2,4-difluorophenyl)-*N*-[[4-(2,2-dimethylpropyl)phenyl]methyl]-*N*-heptylurea). See P. Chang et al., "Current, New and Future Treatments in Dyslipidaemia and Atherosclerosis", Drugs 2000 Jul;60(1); 55-93.

Generally, a total daily dosage of ACAT inhibitor(s) can range from 0.1 to 1000 mg/day in single or 2-4 divided doses.

In another alternative embodiment, the compositions of the present invention can further comprise one or more Cholesteryl Ester Transfer Protein ("CETP") Inhibitors coadministered with or in combination with the bile acid sequestrant (s) and sterol absorption inhibitory of formula (II) discussed above. CETP is responsible for the exchange or transfer of cholesteryl ester carrying HDL and triglycerides in VLDL.

Examples of suitable CETP inhibitors are disclosed in PCT Patent Application No. WO 00/38721 and U.S. Patent No. 6,147,090. Pancreatic cholesteryl ester hydrolase (pCEH) inhibitors such as WAY-121898 also can be coadministered with or in combination with the bile acid sequestrant(s) and sterol absorption inhibitor of formula (II) discussed above.

Generally, a total daily dosage of CETP inhibitor(s) can range from about 0.01 to about 1000 mg/day, and preferably about 0.5 to about 20 mg/kg body weight/day in single or divided doses.

In another alternative embodiment, the compositions of the present invention can further comprise probucol or derivatives thereof (such as AGI-1067 and other derivatives disclosed in U.S. Patents Nos. 6,121,319 and 6,147,250), which can reduce LDL and HDL levels, coadministered with or in combination with the bile acid sequestrant(s) and sterol absorption inhibitor of formula (II) discussed above.

Generally, a total daily dosage of probucol or derivatives thereof can range from 10 to 2000 mg/day, and preferably 500 to 1500 mg/day in single or 2-4 divided doses.

In another alternative embodiment, the compositions of the present invention can further comprise low-density lipoprotein (LDL) receptor activators, coadministered with or in combination with the bile acid sequestrant(s) and sterol absorption inhibitor of formula (II) discussed above. Examples of suitable LDL-receptor activators include HOE-402, an imidazolidinyl-pyrimidine derivative that directly stimulates LDL receptor activity. See M. Huettinger et al., "Hypolipidemic activity of HOE-402 is Mediated by Stimulation of the LDL Receptor Pathway", Arterioscler. Thromb. 1993; 13:1005-12.

Generally, a total daily dosage of LDL receptor activator(s) can range from 1000 mg/day in single or 2-4 divided doses.

In another alternative embodiment, the compositions of the present invention can further comprise fish oil, which contains Omega 3 fatty acids (3-PUFA), which can reduce VLDL and triglyceride levels, coadministered with or in combination with the bile acid sequestrant(s) and sterol absorption inhibitor of formula (II) discussed above. Generally, a total daily dosage of fish oil or Omega 3 fatty acids can range from 1 to 30 grams per day in single or 2-4 divided doses.

In another alternative embodiment, the compositions of the present invention can further comprise natural water soluble fibers, such as psyllium, guar, oat and pectin, which can reduce cholesterol levels, coadministered with or in combination with the bile acid sequestrant(s) and sterol absorption inhibitor of formula (II) discussed above. Generally, a total daily dosage of natural water soluble fibers can range from 0.1 to 10 grams per day in single or 2-4 divided doses.

In another alternative embodiment, the compositions of the present invention can further comprise plant sterols,

plant stanols and/or fatty acid esters of plant stanols, such as sitostanol ester used in BENECOL® margarine, which can reduce cholesterol levels, coadministered with or in combination with the bile acid sequestrant(s) and sterol absorption inhibitor of formula (II) discussed above. Generally, a total daily dosage of plant sterols, plant stanols and/or fatty acid esters of plant stanols can range from 0.5 to 20 grams per day in single or 2-4 divided doses.

In another alternative embodiment, the compositions of the present invention can further comprise antioxidants, such as probucol, tocopherol, ascorbic acid, β-carotene and selenium, or vitamins such as vitamin $B_6$ or vitamin $B_{12}$, coadministered with or in combination with the bile acid sequestrant(s) and sterol absorption inhibitor(s) discussed above. Generally, a total daily dosage of antioxidants or vitamins can range from 0.05 to 10 grams per day in single or 2-4 divided doses.

In another alternative embodiment, the compositions of the present invention can further comprise monocyte and macrophage inhibitors such as polyunsaturated fatty acids (PUFA), thyroid hormones including throxine analogues such as CGS-26214 (a thyroxine compound with a fluorinated ring), gene therapy and use of recombinant proteins such as recombinant apo E, coadministered with or in combination with the bile acid sequestrant(s) and sterol absorption inhibitor of formula (II) discussed above. Generally, a total daily dosage of these agents can range from 0.01 to 1000 mg/day in single or 2-4 divided doses.

Also useful with the present invention are compositions which further comprise hormone replacement agents and compositions. Useful hormone agents and compositions for hormone replacement therapy of the present invention include androgens, estrogens, progestins, their pharmaceutically acceptable salts and derivatives thereof. Combinations of these agents and compositions are also useful.

The dosage of androgen and estrogen combinations vary, desirably from 1 mg to 4 mg androgen and from 1 mg to 3 mg estrogen. Examples include, but are not limited to, androgen and estrogen combinations such as the combination of esterified estrogens (sodium estrone sulfate and sodium equilin sulfate) and methyltestosterone (17-hydroxy-17-methyl-, (17B)- androst-4-en-3-one) available from Solvay Pharmaceuticals, Inc., Marietta, GA, under the tradename Estratest™.

Estrogens and estrogen combinations may vary in dosage from 0,01 mg up to 8 mg, desirably from 0.3 mg to 3.0 mg. Examples of useful estrogens and estrogen combinations include:

(a) the blend of nine (9) synthetic estrogenic substances including sodium estrone sulfate, sodium equilin sulfate, sodium 17 α -dihydroequilin sulfate, sodium 17 α -estradiol sulfate, sodium 17 β -dihydroequilin sulfate, sodium 17 α -dihydroequilenin sulfate, sodium 17 β -dihydroequilenin sulfate, sodium equilenin sulfate and sodium 17 β -estradiol sulfate; available from Duramed Pharmaceuticals, Inc., Cincinnati, OH, under the tradename Cenestin™:
(b) ethinyl estradiol (19-nor-17 α -pregna-1,3,5(10)-trien-20-yne-3,17-diol; available by Schering Plough Corporation, Kenilworth, NJ, under the tradename Estinyl™;
(c) esterified estrogen combinations such as sodium estrone sulfate and sodium equilin sulfate; available from Solvay under the tradename Estratab™ and from Monarch Pharmaceuticals, Bristol, TN, under the tradename Menest™;
(d) estropipate (piperazine estra-1,3,5(10)-trien-17-one, 3-(sulfooxy)-estrone sulfate); available from Pharmacia & Upjohn, Peapack, NJ, under the tradename Ogen™ and from Women First Health Care, Inc., San Diego, CA, under the tradename Ortho-Est™; and
(e) conjugated estrogens (17 α-dihydroequilin, 17 α-estradiol, and 17 β-dihydroequilin); available from Wyeth-Ayerst Pharmaceuticals, Philadelphia, PA, under the tradename Premarin™.

Progestins and estrogens may also be administered with a variety of dosages, generally from 0.05 to 2.0 mg progestin and 0.001 mg to 2 mg estrogen, desirably from 0.1 mg to 1 mg progestin and 0.01 mg to 0.5 mg estrogen. Examples of progestin and estrogen combinations that may vary in dosage and regimen include:

(a) the combination of estradiol (estra-1, 3, 5 (10)-triene-3, 17 β-diol hemihydrate) and norethindrone (17 β-acetoxy-19-nor-17 α-pregn-4-en-20-yn-3-one); which is available from Pharmacia & Upjohn, Peapack, NJ, under the tradename Activella™;
(b) the combination of levonorgestrel (d(-)-13 β-ethyl-17 α-ethinyl-17 β-hydroxygon- 4-en-3-one) and ethinyl estradial; available from Wyeth-Ayerst under the tradename Alesse, from Watson Laboratories, Inc., Corona, CA, under the tradenames Levora and Trivora, Monarch Pharmaceuticals, under the tradename Nordette™, and from Wyeth-Ayerst under the tradename Triphasil™;
(c) the combination of ethynodiol diacetate (19-nor-17 α-pregn-4-en-20-yne-3 β, 17-diol diacetate) and ethinyl estradiol; available from G.D. Searle & Co., Chicago, IL, under the tradename Demulen™ and from Watson under the tradename Zovia™;
(d) the combination of desogestrel (13-ethyl-11- methylene-18,19-dinor-17 α-pregn- 4-en- 20-yn-17-ol) and ethinyl estradiol; available from Organon under the tradenames Desogen™ and Mircette™, and from Ortho-McNeil Pharmaceutical Raritan, NJ, under the tradename Ortho-Cept™;

(e) the combination of norethindrone and ethinyl estradiol; available from Parke-Davis, Morris Plains, NJ, under the tradenames Estrostep™ and fernhrt™, from Watson under the tradenames Microgestin™, Necon™, and Tri-Norinyl™, from Ortho-McNeil under the tradenames Modicon™ and Ortho-Novum™, and from Warner Chilcott Laboratories, Rockaway, NJ, under the tradename Ovcon™;

(f) the combination of norgestrel ( (±)-13-ethyl-17-hydroxy-18, 19-dinor-17 α-preg-4-en-20-yn-3-one) and ethinyl estradiol; available from Wyeth-Ayerst under the tradenames Ovral™ and Lo/Ovral™, and from Watson under the tradenames Ogestrel™ and Low-Ogestrel™;

(g) the combination of norethindrone, ethinyl estradiol, and mestranol (3-methoxy-19-nor-17 α-pregna-1,3,5(10)-trien-20-yn-17-ol); available from Watson under the tradenames Brevicon™ and Norinyl™;

(h) the combination of 17 β-estradiol (estra-1,3,5(10)-triene-3,17 β-diol) and micronized norgestimate (17 α-17-(Acetyloxyl)-13-ethyl-18,19-dinorpregn-4-en-20-yn-3-one3-oxime); available from Ortho-McNeil under the tradename Ortho-Prefest™;

(i) the combination of norgestimate (18,19-dinor-17-pregn-4-en-20-yn-3-one, 17-(acetyloxy)-13-ethyl-,oxime, (17 (α)-(+)-) and ethinyl estradiol; available from Ortho-McNeil under the tradenames Ortho Cyclen™ and Ortho Tri-Cyclen™; and

(j) the combination of conjugated estrogens (sodium estrone sulfate and sodium equilin sulfate) and medroxyprogesterone acetate (20-dione, 17-(acetyloxy)-6-methyl-, (6(α))- pregn-4-ene-3); available from Wyeth-Ayerst under the tradenames Premphase™ and Prempro™.

In general, a dosage of progestins may vary from .05 mg to 10 mg or up to 200 mg if microsized progesterone is administered. Examples of progestins include norethindrone; available from ESI Lederle, Inc., Philadelphia, PA, under the tradename Aygestin™, from Ortho-McNeil under the tradename Micronor™, and from Watson under the tradename Nor-QD™; norgestrel; available from Wyeth-Ayerst under the tradename Ovrette™; micronized progesterone (pregn-4-ene-3, 20-dione); available from Solvay under the tradename Prometrium™; and medroxyprogesterone acetate; available from Pharmacia & Upjohn under the tradename Provera™.

The compositions or therapeutic combinations of the present invention can further comprise one or more obesity control medications. Useful obesity control medications include drugs that reduce energy intake or suppress appetite, drugs that increase energy expenditure and nutrient-partitioning agents. Suitable obesity control medications include noradrenergic agents (such as diethylpropion, mazindol, phenylpropanolamine, phentermine, phendimetrazine, phendamine tartrate, methamphetamine, phendimetrazine and tartrate); serotonergic agents (such as sibutramine, fenfluramine, dexfenfluramine, fluoxetine, fluvoxamine and paroxtine); thermogenic agents (such as ephedrine, caffeine, theophylline, and selective β3-adrenergic agonists); alpha-blocking agents; kainite or AMPA receptor antagonists; leptin-lipolysis stimulated receptors; phosphodiesterase enzyme inhibitors; compounds having nucleotide sequences of the mahogany gene; fibroblast growth factor-10 polypeptides; monoamine oxidase inhibitors (such as befloxatone, moclobemide, brofaromine, phenoxathine, esuprone, befol, toloxatone, pirlindol, amiflamine, sercloremine, bazinaprine, lazabemide, milacemide and caroxazone); compounds for increasing lipid metabolism (such as evodiamine compounds); and lipase inhibitors (such as orlistat). Generally, a total dosage of the above-described obesity control medications can range from 1 to 3,000 mg/day, desirably from 1 to 1,000 mg/day and more desirably from 1 to 200 mg/day in single or 2-4 divided doses.

The compositions or therapeutic combinations of the present invention can further comprise one or more blood modifiers which are chemically different from the substituted azetidinone and substituted β-lactam compounds i.e. compounds II above) and the bile acid sequestrants discussed above, for example, they contain one or more different atoms, have a different arrangement of atoms or different number of one or more atoms than the sterol absorption inhibitor of formula (II) or bile acid sequestrants discussed above. Useful blood modifiers include anti-coagulants (argatroban, bivalirudin, dalteparin sodium, desirudin, dicumarol, lyapolate sodium, nafamostat mesylate, phenprocoumon, tinzaparin sodium, warfarin sodium); antithrombotic (anagrelide hydrochloride, bivalirudin, cilostazol, dalteparin sodium, danaparoid sodium, dazoxiben hydrochloride, efegatran sulfate, enoxaparin sodium, fluretofen, ifetroban, ifetroban sodium, lamifiban, lotrafiban hydrochloride, napsagatran, orbofiban acetate, roxifiban acetate, sibrafiban, tinzaparin sodium, trifenagrel, abciximab, zolimomab aritox); fibrinogen receptor antagonists (roxifiban acetate, fradafiban, orbofiban, lotrafiban hydrochloride, tirofiban, xemilofiban, monoclonal antibody 7E3, sibrafiban); platelet inhibitors (cilostazol, clopidogrel bisulfate, epoprostenol, epoprostenol sodium, ticlopidine hydrochloride, aspirin, ibuprofen, naproxen, sulindae, idomethacin, mefenamate, droxicam, diclofenac, sulfinpyrazone, piroxicam, dipyridamole); platelet aggregation inhibitors (acadesine, beraprost, beraprost sodium, ciprostene calcium, itazigrel, lifarizine, lotrafiban hydrochloride, orbofiban acetate, oxagrelate, fradafiban, orbofiban, tirofiban, xemilofiban); hemorrheologic agents (pentoxifylline); lipoprotein associated coagulation inhibitors; Factor VIIa inhibitors (4H-31-benzoxazin-4-ones, 4H-3,1-benzoxazin-4-thiones, quinazolin-4-ones, quinazolin-4-thiones, benzothiazin-4-ones, imidazolyl-boronic acid-derived peptide analogues TFPI-derived peptides, naphthalene-2-sulfonic acid {1-[3-(aminoiminomethyl)-benzyl]-2-oxo-pyrrolidin-3-(S)-yl) amide trifluoroacetate,

dibenzofuran-2-sulfonic acid {1-[3-(aminomethyl)benzyl]-5-oxo-pyrrolidin-3-yl}-amide, tolulene-4-sulfonic acid {1-[3-(aminoiminomethyl)-benzyl]-2-oxo-pyrrolidin-3-(S)-yl}-amide trifluoroacetate, 3,4-dihydro-1H-isoquinoline-2-sulfonic acid {1-[3-(aminoiminomethyl}-benzy)]-2-oxo-pyrrolin-3-(S)-yl}-amide trifluoroacetate); Factor Xa inhibitors (disubstituted pyrazolines, disubstituted triazolines, substituted n-[(aminoiminomethyl)phenyl] propylamides, substituted n-[(aminomethyl)phenyl] propylamides, tissue factor pathway inhibitor (TFPI), low molecular weight heparins, heparinoids, benzimidazolines, benzoxazolinones, benzopiperazinones, indanones, dibasic (amidinoaryl) propanoic acid derivatives, amidinophenyl-pyrrolidines, amidinophenyl-pyrrolines, amidinophenyl-isoxazolidines, amidinoindoles, amidinoazoles, bis-arlysulfonylaminobenzamide derivatives, peptidic Factor Xa inhibitors).

Useful cardiovascular agents are selected from calcium channel blockers (clentiazem maleate, amiodipine besylate, isradipine, nimodipine, felodipine, nilvadipine, nifedipine, teludipine hydrochloride, diltiazem hydrochloride, belfosdil, verapamil hydrochloride, fostedil); adrenergic blockers (fenspiride hydrochloride, labetalol hydrochloride, proroxan, alfuzosin hydrochloride, acebutolol, acebutolol hydrochloride, alprenolol hydrochloride, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol sulfate, labetalol hydrochloride, levobetaxolol hydrochloride, levobunolol hydrochloride, metalol hydrochloride, metoprolol, metoprofol tartrate, nadolol, pamatolol sulfate, penbutolol sulfate, practolol, propranolol hydrochloride, sotalol hydrochloride, timolol, timolol maleate, tiprenolol hydrochloride, tolamolol, bisoprolol, bisoprolol fumarate, nebivolol); adrenergic stimulants; angiotensin converting enzyme (ACE) inhibitors (benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride, spiraprilat, teprotide, enalapril maleate, lisinopril, zofenopril calcium, perindopril erbumine); antihypertensive agents (althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amlodipine besylate, amlodipine maleate, bevantolol hydrochloride); angiotensin II receptor antagonists (candesartan, irbesartan, losartan potassium, candesartan cilexetil, telmisartan); anti-anginal agents (amiodipine besylate, amlodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride); coronary vasodilators (fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate, terodiline hydrochloride, tolamolol, verapamil); diuretics (the combination product of hydrochlorothiazide and spironolactone and the combination product of hydrochlorothiazide and triamterene).

The compositions or therapeutic combinations of the present invention can further comprise one or more antidiabetic medications for reducing blood glucose levels in a human. Useful antidiabetic medications include drugs that reduce energy intake or suppress appetite, drugs that increase energy expenditure and nutrient-partitioning agents. Suitable antidiabetic medications include sulfonylurea (such as acetohexamide, chlorpropamide, gliamilide, gliclazide, glimepiride, glipizide, glyburide, glibenclamide, tolazamide, and tolbutamide), meglitinide (such as repaglinide and nateglinide), biguanide (such as metformin and buformin), alpha-glucosidase inhibitor (such as acarbose, miglitol, camiglibose, and voglibose), certain peptides (such as amlintide, pramlintide, exendin, and GLP-1 agonistic peptides), and orally administrable insulin or insulin composition for intestinal delivery thereof. Generally, a total dosage of the above-described antidiabetic medications can range from 0.1 to 1,000 mg/day in single or 2-4 divided doses.

Mixtures of any of the pharmacological or therapeutic agents described above can be used in the compositions and therapeutic combinations of the present invention.

The compositions and therapeutic combinations of the present invention can be administered to a mammal in need of such treatment in a therapeutically effective amount to treat vascular conditions such as atherosclerosis, hypertipidaemia (including hypercholesterolaemia, hypertriglyceridaemia, sitosterolemia), diabetes, obesity. The compositions can be administered by any suitable means which produce contact of these compounds with the site of action in the body, for example in the plasma, liver or ileum of a mammal or mammal.

The daily dosage for the various compositions and therapeutic combinations described above can be administered to a patient in a single dose or in multiple subdoses, as desired. Subdoses can be administered 2 to 6 times per day, for example. Sustained release dosages can be used. Where the bile acid sequestrant(s) and sterol absorption inhibitor of formula (II) are administered in separate dosages, the number of doses of each component given per day may not necessarily be the same, e.g., one component may have a greater duration of activity and will therefore need to be administered less frequently.

Since the present invention relates to reducing the plasma sterol (especially cholesterol) concentrations or

levels by treatment with a combination of active ingredients wherein the active ingredients may be administered separately, the invention also relates to combining separate pharmaceutical compositions in kit form. That is, a kit is contemplated wherein two separate units are combined: a pharmaceutical composition comprising at least one bile acid sequestrant and a separate pharmaceutical composition comprising at least one sterol absorption inhibitor as described above. The kit will preferably include directions for the administration of the separate components. The kit form is particularly advantageous when the separate components must be administered in different dosage forms (e.g., oral and parenteral) or are administered at different dosage intervals.

The pharmaceutical treatment compositions and therapeutic combinations of the present invention can further comprise one or more pharmaceutically acceptable carriers, one or more excipients and/or one or more additives. Examples of pharmaceutically acceptable carriers include solids and/or liquids such as ethanol, glycerol, water. The amount of carrier in the treatment composition can range from 5 to 99 weight percent of the total weight of the treatment composition or therapeutic combination. Suitable pharmaceutically acceptable excipients and additives include non-toxic compatible fillers, binders such as starch, disintegrants, buffers, preservatives, anti-oxidants, lubricants, flavorings, thickeners, coloring agents, emulsifiers. The amount of excipient or additive can range from 0.1 to 90 weight percent of the total weight of the treatment composition or therapeutic combination. One skilled in the art would understand that the amount of carrier(s), excipients and additives (if present) can vary.

The treatment compositions of the present invention can be administered in any conventional dosage form, preferably an oral dosage form such as a capsule, tablet, powder, cachet, suspension or solution. The formulations and pharmaceutical compositions can be prepared using conventional pharmaceutical acceptable and conventional techniques. Several examples of preparation of dosage formulations are provided below.

The following formulations exemplify some of the dosage forms of this invention. In each formulation, the term "Active Compound I" designates a substituted azetidinone compound or β-lactam compound of formula (II) and the term "Active Compound II" designates a bile acid sequestrant described herein above.

EXAMPLE (Reference Example - not within the scope of the invention)

Tablets.

| No. | Ingredient | mg/tablet |
|---|---|---|
| 1 | Active Compound I | 10 |
| 2 | Lactose monohydrate NF | 55 |
| 3 | Microcrystalline cellulose | 20 |
| 4 | Povidone (K29-32) USP | 4 |
| 5 | Croscarmellose sodium NF | 8 |
| 6 | Sodium lauryl sulfate | 2 |
| 7 | Magnesium stearate NF | 1 |
| | Total | 100 |

In the present invention, the above-described tablet can be coadministered with a tablet, capsule, etc. comprising a dosage of Active Compound II, for example a tablet of QUESTRAN® cholestyramine as described above.

Method of Manufacture

Mix Item No. 4 with purified water in suitable mixer to form binder solution. Spray the binder solution and then water over Items 1, 2, 6 and a portion of Item 5 in a fluidized bed processor to granulate the ingredients. Continue fluidization to dry the damp granules. Screen the dried granules and blend with Item No. 3 and the remainder of Item 5. Add Item No. 7 and mix. Compress the mixture to appropriate size and weight on a suitable tablet machine.

For coadministration in separate tablets or capsules, representative formulations comprising a cholesterol absorption inhibitor such as are discussed above are well known in the art and representative formulations comprising a bile acid sequestrant such as are discussed above are well known in the art. It is contemplated that where the two active ingredients are administered as a single composition, the dosage forms disclosed above for substituted azetidinone or β-lactam compounds of formula II may readily be modified using the knowledge of one skilled in the art.

The treatment compositions and therapeutic combinations of the present invention can inhibit the intestinal absorption of cholesterol in mammals, and can be useful in the treatment and/or prevention of vascular conditions, such

as atherosclerosis, hypercholesterolemia and sitosterolemia, stroke, diabetes, obesity and lowering of plasma levels of cholesterol in mammals, in particular in mammals.

In another embodiment of the present invention, the compositions and therapeutic combinations of the present invention can inhibit sterol absorption or reduce plasma concentration of at least one sterol selected from the group consisting of phytosterols (such as sitosterol, campesterol, stigmasterol and avenosterol), 5α-stanols (such as cholestanol, 5α-campestanol, 5α-sitostanol), cholesterol and mixtures thereof. The plasma concentration can be reduced by administering to a mammal in need of such treatment an effective amount of at least one treatment composition or therapeutic combination comprising at least one bile acid sequestrant and at least one sterol absorption inhibitor described above. The reduction in plasma concentration of sterols can range from about 1 to about 70 percent, and preferably about 10 to about 50 percent. Methods of measuring serum total blood cholesterol and total LDL cholesterol are well known to those skilled in the art and for example include those disclosed in PCT WO 99/38498 at page 11. Methods of determining levels of other sterols in serum are disclosed in H. Gylling et al., "Serum Sterols During Stanol Ester Feeding in a Mildly Hypercholesterolemic Population", J. Lipid Res. 40: 593-600 (1999).

Unless otherwise indicated, all parts and percentages in the following examples, as well as throughout the specification, are by weight.

**EXAMPLE 1** (Reference Example not within the scope of the invention)

To follow the movement of lipids across the intestinal wall leading to its subsequent appearance in the lymph, blood and liver, a short term hamster model which tracked a bolus of gavaged radiolabeled lipid was used. Free cholesterol absorption was examined in this hamster model to explore whether the cholesterol absorption inhibitor compound of Formula (XII):

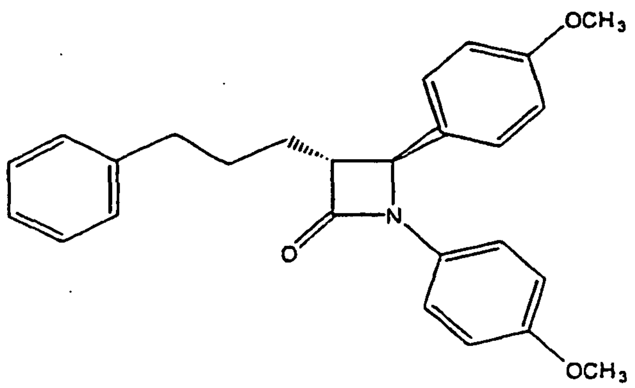

(XII)

in combination with the bile acid sequestrant cholestyramine would have additive efficacy. Compound XII can be prepared as shown in Example 9 of U.S. Patent No. 5,688,787.

Male Golden Syrian hamsters were fed a diet containing 0.5% cholesterol overnight and divided into groups of 5 animals. In the morning they were gavaged with 1 uCi of [$^{14}$C]-cholesterol with 1 mg of unlabelled cholesterol (NEN/Dupont) in 0.2 ml of corn oil, one hour after they were gavaged with corn oil as a Control, compound of Formula (XII) (3mg/kg of body weight), cholestyramine (1g/kg of body weight), or the compound of Formula (XII) combined with cholestyramine as described in Table 1 below. Two hours later, blood and liver samples were collected from each hamster. The blood was centrifuged for 20 minutes at 3000 rpm and the plasma was removed. Aliquots of the plasma and liver were analyzed for [$^{14}$C] radioactivity. Radioactive content was determined using a Beckman LSC (liquid simulation counter).

**Table 1**

| Group | Plasma [$^{14}$C]-cholesterol (Decays Per Minute (DPM)/milliter of plasma) | Liver [$^{14}$C]-cholesterol (DPM/gram of liver) |
|---|---|---|
| Control | 4945 ± 644 | 8035 ± 1611 |
| Compound XII (3mg/kg of body weight) | 1438 ± 455 (-71 %) | 3755 ± 923 (-53%) |

(continued)

| Group | Plasma [$^{14}C$]-cholesterol (Decays Per Minute (DPM)/milliter of plasma) | Liver [$^{14}C$]-cholesterol (DPM/gram of liver) |
|---|---|---|
| Cholestyramine (1g/kg of body weight) | 836 ± 320 (-83%) | 3300 ± 1252 (-60%) |
| Compound XII (3mg/kg) + Cholestyramine (1g/kg of body weight) | 478 ± 101 (-90%) | 1196 ± 247 (-85%) |

Administration of the specified dosage of Compound XII alone in male Golden Syrian hamsters reduced plasma and liver [$^{14}C$]-cholesterol levels by 71 % and 53%, respectively (see Table 1). Administration of the specified dosage of cholestyramine alone reduced plasma and liver [$^{14}C$]-cholesterol levels by 83% and 60%, respectively. The specified combination of Compound XII and cholestyramine resulted in reductions in plasma and hepatic (liver) [$^{14}C$]-cholesterol levels by 90% and 85%, respectively (see Table 1). These results indicate that the combination of the cholesterol absorption inhibitor, Compound XII, and the bile acid sequestrant, cholestyramine, may have additional effects on treating hypercholesterolemia by reducing both plasma and hepatic cholesterol levels.

## EXAMPLE 2

PREPARATION OF COMPOUND OF FORMULA (II)

Step 1): To a solution of (S)-4-phenyl-2-oxazolidinone (41 g, 0.25 mol) in $CH_2Cl_2$ (200 ml), was added 4-dimethylaminopyridine (2.5 g, 0.02 mol) and triethylamine (84.7 ml, 0.61 mol) and the reaction mixture was cooled to 0°C. Methyl-4-(chloroformyl)butyrate (50 g, 0.3 mol) was added as a solution in $CH_2Cl_2$ (375 ml) dropwise over 1 h, and the reaction was allowed to warm to 22°C. After 17 h, water and $H_2SO_4$ (2N, 100 ml), was added the layers were separated, and the organic layer was washed sequentially with NaOH (10%), NaCl (sat'd) and water. The organic layer was dried over $MgSO_4$ and concentrated to obtain a semicrystalline product.

Step 2): To a solution of $TiCl_4$ (18.2 ml, 0.165 mol) in $CH_2Cl_2$ (600 ml) at 0°C, was added titanium isopropoxide (16.5 ml, 0.055 mol). After 15 min, the product of Step 1 (49.0 g, 0.17 mol) was added as a solution in $CH_2Cl_2$ (100 ml). After 5 min., diisopropylethylamine (DIPEA) (65.2 ml, 0.37 mol) was added and the reaction mixture was stirred at 0°C for 1 h, the reaction mixture was cooled to -20°C, and 4-benzyloxybenzylidine(4-fluoro)aniline (114.3 g, 0.37 mol) was added as a solid. The reaction mixture was stirred vigorously for 4 h at -20°C, then acetic acid was added as a solution in $CH_2Cl_2$ dropwise over 15 min, the reaction mixture was allowed to warm to 0°C, and $H_2SO_4$ (2N) was added. The reaction mixture was stirred an additional 1 h, the layers were separated, washed with water, separated and the organic layer was dried. The crude product was crystallized from ethanol/water to obtain the pure intermediate.

Step 3): To a solution of the product of Step 2 (8.9 g, 14.9 mmol) in toluene (100 ml) at 50°C, was added N,O-bis(trimethylsilyl)acetamide (BSA) (7.50 ml, 30.3 mmol). After 0.5 h, solid TBAF (0.39 g, 1.5 mmol) was added and the reaction mixture stirred at 50°C for an additional 3 h. The reaction mixture was cooled to 22°C, $CH_3OH$ (10 ml), was added. The reaction mixture was washed with HCl (1 N), $NaHCO_3$ (1 N) and NaCl (sat'd.), and the organic layer was dried over $MgSO_4$.

Step 4): To a solution of the product of Step 3 (0.94 g, 2.2 mmol) in $CH_3OH$ (3 ml), was added water (1 ml) and $LiOH \cdot H_2O$ (102 mg, 2.4 mmole). The reaction mixture was stirred at 22°C for 1 h and then additional $LiOH \cdot H_2O$ (54 mg, 1.3 mmole) was added. After a total of 2 h, HCl (1 N) and EtOAc was added, the layers were separated, the organic layer was dried and concentrated in *vacuo.* To a solution of the resultant product (0.91 g, 2.2 mmol) in $CH_2Cl_2$ at 22°C, was added ClCOCOCl (0.29 ml, 3.3 mmol) and the mixture stirred for 16 h. The solvent was removed in *vacuo.*

Step 5): To an efficiently stirred suspension of 4-fluorophenylzinc chloride (4.4 mmol) prepared from 4-fluorophenylmagnesium bromide (1 M in THF, 4.4 ml, 4.4 mmol) and $ZnCl_2$ (0.6 g, 4.4 mmol) at 4°C, was added tetrakis (triphenylphosphine)palladium (0.25 g, 0.21 mmol) followed by the product of Step 4 (0.94 g, 2.2 mmol) as a solution in THF (2 ml). The reaction was stirred for 1 h at 0°C and then for 0.5 h at 22°C. HCl (1 N, 5 ml) was added and the mixture was extracted with EtOAc. The organic layer was concentrated to an oil and purified by silica gel chromatography to obtain 1-(4-fluorophenyl)-4(S)-(4-hydroxyphenyl)-3(R)-(3-oxo-3-phenylpropyl)-2-azetidinone:
HRMS calc'd for $C_{24}H_{19}F_2NO_3$ = 408.1429, found 408.1411.

Step 6): To the product of Step 5 (0.95 g, 1.91 mmol) in THF (3 ml), was added (R)-tetrahydro-1-methyl-3,3-diphenyl-1 H,3H-pyrrolo-[1,2-c][1,3,2] oxazaborole (120 mg, 0.43 mmol) and the mixture was cooled to -20ºC. After 5 min, borohydride-dimethylsulfide complex (2M in THF, 0.85 ml, 1.7 mmol) was added dropwise over 0.5 h. After a total of 1.5 h , $CH_3OH$ was added followed by HCl (1 N) and the reaction mixture was extracted with EtOAc to obtain 1-(4-fluorophenyl)-3(R)-[3(S)-(4-fluorophenyl)-3-hydroxypropyl)]-4(S)-[4-(phenylmethoxy)phenyl]-2-azetidinone (compound 6A-1) as an oil. $^1H$ in $CDCl_3$ d H3 = 4.68. J = 2.3 Hz. Cl ($M^+H$) 500.

Use of (S)-tetra-hydro-1-methyl-3,3-diphenyl-1H,3H-pyrrolo-[1,2-c][1,3,2] oxazaborole gives the corresponding 3(R)-hydroxypropyl azetidinone (compound 6B-1). $^1H$ in $CDCl_3$ d H3 = 4.69. J = 2.3 Hz. Cl ($M^+H$) 500.

To a solution of compound 6A-1 (0.4 g, 0.8 mmol) in ethanol (2 ml), was added 10% Pd/C (0.03 g) and the reaction mixture was stirred under a pressure (60 psi) of $H_2$ gas for 16 h. The reaction mixture was filtered and the solvent was concentrated to obtain compound 6A. Mp 164-166°C; Cl ($M^+H$) 410. $[\alpha]_D^{25}$ = -28.1° (c 3, $CH_3OH$). Elemental analysis calc'd for $C_{24}H_{21}F_2NO_3$: C 70.41; H 5.17; N 3.42; found C 70.25; H 5.19; N 3.54.

Similarly treat compound 6B-1 to obtain compound 6B.
Mp 129.5-132.5°C; Cl ($M^+H$) 410. Elemental analysis calc'd for $C_{24}H_{21}F_2NO_3$: C 70.41; H 5.17; N 3.42; found C 70.30; H 5.14; N3.52.

Step 6' (Alternative): To a solution of the product of Step 5 (0.14 g, 0.3 mmol) in ethanol (2 ml), was added 10% Pd/C (0.03 g) and the reaction was stirred under a pressure (60 psi) of $H_2$ gas for 16 h. The reaction mixture was filtered and the solvent was concentrated to afford a 1:1 mixture of compounds 6A and 6B.

## Claims

**1.** A composition comprising:

(a) at least one of cholestyramine, colestipol, colesevelam hydrochloride, and mixtures thereof, and
(b) at least one sterol absorption inhibitor represented by the following formula (II)

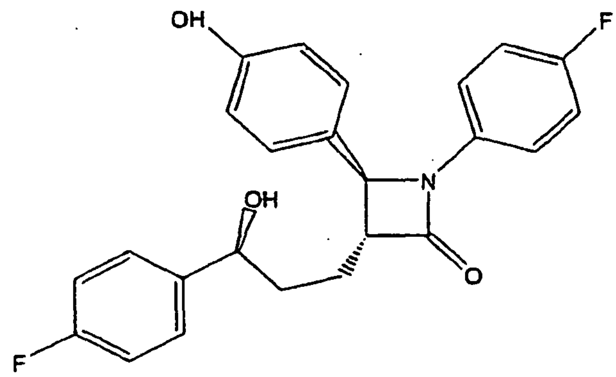

(II)

or a pharmaceutically acceptable salt or solvate thereof.

**2.** A therapeutic combination comprising:

(a) a first amount of at least one of cholestyramine, colestipol, colesevelam hydrochloride, and mixtures thereof; and
(b) a second amount of at least one sterol absorption inhibitor represented by the following formula (II)

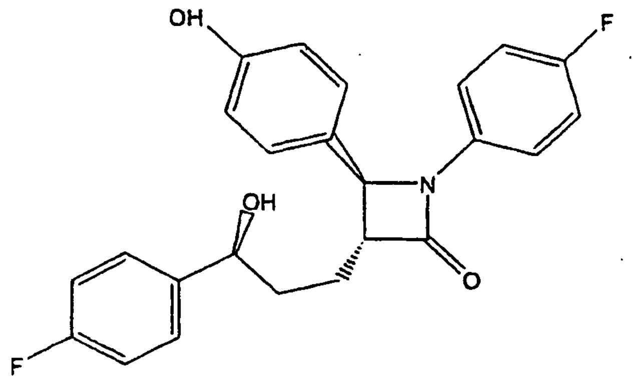

(II)

or a pharmaceutically acceptable salt or solvate thereof.

wherein the first amount and the second amount together comprise a therapeutically effective amount for for the treatment or prevention of diabetes, obesity or a vascular condition selected from hyperlipidaemia, atherosclerosis, hypercholesterolemia.

**3.** The composition or therapeutic combination according to any of claims 1 or 2, wherein the at least of cholestyramine, colestipol, colesevelam hydrochloride, and mixtures thereof comprises cholestyramine.

**4.** The composition or therapeutic combination according to claim 3, wherein the at least one of cholestyramine, colestipol, colesevelam hydrochloride, and mixtures thereof comprises colestipol.

**5.** The composition or therapeutic combination according to any of claims 1 or 2, wherein the at least one of cholestyramine, colestipol, colesevelam hydrochloride, and mixtures thereof to be administered to a mammal in an amount ranging from 1 to 50 grams of bile acid sequestrant per day.

**6.** The composition or therapeutic combination according to any of claims 1 or 2, wherein the at least one sterol absorption inhibitor is to be administered to a mammal in an amount ranging from 0.1 to 1000 milligrams of sterol absorption inhibitor per day.

**7.** The composition or therapeutic combination according to any of claims 1 or 2, further comprising at least one compound selected from the group consisting of lovastatin, pravastatin, fluvastatin, simvastatin, atorvastatin, cerivastatin, CI-981, pitavastatin, L-659,699, squalestatin 1, NB-598 and DMP-565

**8.** The composition or therapeutic combination according to claim 7, wherein the at least one compound is selected from the group consisting of lovastatin, pravastatin, fluvastatin simvastatin, atorvastatin, cerivastatin and mixtures thereof.

**9.** The composition or therapeutic combination according to claim 8, wherein the at least one compound is simvastatin.

**10.** The composition or therapeutic combination according to any of claims 1 or 2, further comprising at least one lipid lowering agent selected from the group consisting of clofibrate, gemfibrozil, ciprofibrate, bezafibrate, clinofibrate, binifibrate, lifibrol, fenofibrate, troglitazone, rosiglitazone, pioglitazone, ciglitazone, englitazone darglitazone, BRL 49653, C.A:S: Registry No. 317318-32-4, GW2331, nicotinic acid, niceritrol, nicofuranose, acipimox, avasimibe, HL-004, lecimibide, probucol, AGI-1067, HOE-402, psyllium, guar, oat, pectin and sitostanol ester.

**11.** The composition or therapeutic combination according to any of claims 1 or 2, further comprising at least one additive selected from the group consisting of probucol, tocopherol, ascorbic acid, β-carotene, selenium, vitamin $B_6$, vitamin $B_{12}$ androgens and combinations thereof, estrogens and combinations thereof, progestins, a combination of progestins and estrogens, diethylpropion, mazindol, phenylpropanolamine, phentermine, phendimetrazine, phendamine tartrate, methamphetamine, tartrate, sibutramine, fenfluroamine, dexfenfluramine, fluoxetine, fluvoxamine, paroxtine, fefloxatone, moclobemide, brofaromine, phenoxathine, esuprone, befol, toloxatone, pirlindol, amiflamine, sercloremine, bazinaprine, lazabemide, milacemide, caroxazone, orlistat, argatroban, bivalirudin, dalteparin sodium, desirudin, dicumarol, lyapolate sodium, nafomastat mesylate, phenprocoumon, tinzaparin sodium, warfarin sodium, anagrelide hydrochloride, cilostazol, danaparoid sodium, dazoxiben hydrochloride, efegatran sulfate, enoxaparin sodium, fluretofen, ifetroban, ifetroban sodium, lamifiban, lotrafiban hydrochloride, napsagatran, orbofiban acetate, roxifiban acetate, sibrafiban, trifenagrel, abciximab, zolimomab aritox, tirofiban, xemilofiban, monoclonal antibody 7E3, clopidogrel bisulfate, epoprostenol, epoprostenol sodium, ticlopidine hydrochloride, aspirin, ibuprofen, naproxen, sulindae, idomethacin, mefenamate, droxicam, diclofenac, sulfinpyrazone, piroxicam, dipyridamole, acadesine, beraprost, beraprost sodium, ciprostene calcium, itazigrel, lifarizine, oxagrelate, fradafiban, orbofiban, naphtalene-2-sulfonic acid {1-[3-(aminoiminomethyl)-benzyl]-2-oxopyrrolidin-3-(S)-yl} amide trifluoroacetate, dibenzofuran-2-sulfonic acid {1-[3-(aminomethyl)benzyl]-5-oxo-pyrrolidin-3-yl} amide, toluene-4-sulfonic acid {1-[3-(aminoiminomethyl)-benzyl]-2-oxo-pyrrolidin-3-(S)-yl} amide trifluoroacetate, 3,4-dihydro-1 H-isoquinoline-2-sulfonic acid {1-[3-(aminoiminomethyl)-benzyl]-2-oxopyrrolidin-3-(S)-yl} amide trifluoroacetate, clentiazem maleate, amiodipine besylate, isradipine, nimodipine, felodipine, nilvadipine, nifedipine, teludipine hydrochloride, diltiazem hydrochloride, belfosdil, verapamil hydrochloride, fostedil; fenspiride hydrochloride, labetalol hydrochloride, proroxan, alfuzosin hydrochloride, acebutolol, acebutolol hydrochloride, alprenolol hydrochforide, atenolol, bunolol hydrochloride, carteolol hydrochloride, celiprolol hydrochloride, cetamolol hydrochloride, cicloprolol hydrochloride, dexpropranolol hydrochloride, diacetolol hydrochloride, dilevalol hydrochloride, esmolol hydrochloride, exaprolol hydrochloride, flestolol sulfate, labetalol hydrochloride, levobetaxolol hydrochloride, levobunolol hydrochloride, metalol hydrochloride, metoprolol, metoprolol tartrate, nadolol, pamatolol sulfate, penbutolol sulfate, practolol, propranolol hydrochloride, sotalol hydrochloride, timolol, timolol maleate, tiprenolol hydrochloride, tolamolol, bisoprolol, bisoprolol fumarate, nebivolol; benazepril hydrochloride, benazeprilat, captopril, delapril hydrochloride, fosinopril sodium, libenzapril, moexipril hydrochloride, pentopril, perindopril, quinapril hydrochloride, quinaprilat, ramipril, spirapril hydrochloride, spiraprilat, teprotide, enalapril maleate, lisinopril, zofenopril calcium, perindopril erbumine, althiazide, benzthiazide, captopril, carvedilol, chlorothiazide sodium, clonidine hydrochloride, cyclothiazide, delapril hydrochloride, dilevalol hydrochloride, doxazosin mesylate, fosinopril sodium, guanfacine hydrochloride, methyldopa, metoprolol succinate, moexipril hydrochloride, monatepil maleate, pelanserin hydrochloride, phenoxybenzamine hydrochloride, prazosin hydrochloride, primidolol, quinapril hydrochloride, quinaprilat, ramipril, terazosin hydrochloride, candesartan, candesartan cilexetil, telmisartan, amiodipine besylate, amiodipine maleate, bevantolol hydrochloride, candesartan, irbesartan, losartan potassium, candesartan cilexetil, telmisartan amlodipine besylate, amiodipine maleate, betaxolol hydrochloride, bevantolol hydrochloride, butoprozine hydrochloride, carvedilol, cinepazet maleate, metoprolol succinate, molsidomine, monatepil maleate, primidolol, ranolazine hydrochoride, tosifen, verapamil hydrochloride, fostedil, azaclorzine hydrochloride, chromonar hydrochloride, clonitrate, diltiazem hydrochloride, dipyridamole, droprenilamine, erythrityl tetranitrate, isosorbide dinitrate, isosorbide mononitrate, lidoflazine, mioflazine hydrochloride, mixidine, molsidomine, nicorandil, nifedipine, nisoldipine, nitroglycerine, oxprenolol hydrochloride, pentrinitrol, perhexiline maleate, prenylamine, propatyl nitrate, terodiline hydrochloride, tolamolol, veraparnil; the combination product of hydrochlorothiazide and spironolactone, the combination product of hydrochlorothiazide and triamterene-, acetohexamide, chlorpropamide, gliamilide, gliclazide, glimepiride, glipizide, glyburide, glibenclamide, tolazamide, tolbutamide, repaglinide and nateglinide, metformin and buformin, acarbose, miglitol, camiglibose, voglibose, amlintide, pramlintide, and exendin.

**12.** A pharmaceutical composition for the treatment or prevention of hyperlipidemia, atherosclerosis, diabetes, obesity or hypercholesterolemia, comprising a therapeutically effective amount of the composition of claim 1 and a pharmaceutically acceptable carrier.

**13.** Use of a composition according to any one of claims 1-12 for the manufacture of a medicament for the prevention or treatment of a hyperlipidemia, atherosclerosis, diabetes, obesity, or hypercholesterolemia.

**14.** Use according to claim 13, for the treatment or prevention of hyperlipidemia.

**15.** The therapeutic combination according to claim 2, wherein the at least one of cholestyramine, colestipol, colesevelam hydrochloride, and mixtures thereof is administered concomitantly with the at least one sterol absorption inhibitor represented by Formula (II),

**16.** The therapeutic combination according to claim 2, wherein the at least one of cholestyramine, colestipol, colesevelam hydrochloride, and mixtures thereof, and the sterol absorption inhibitor represented by Formula (II) are present in separate treatment compositions,

**Patentansprüche**

1. Zusammensetzung, umfassend:

(a) zumindest eines von Cholestyramin, Colestipol, Colesevelam hydrochlorid und Mischungen hiervon, und
(b) zumindest einen Sterolabsorptionsinhibitor, repräsentiert durch die folgende Formel (II):

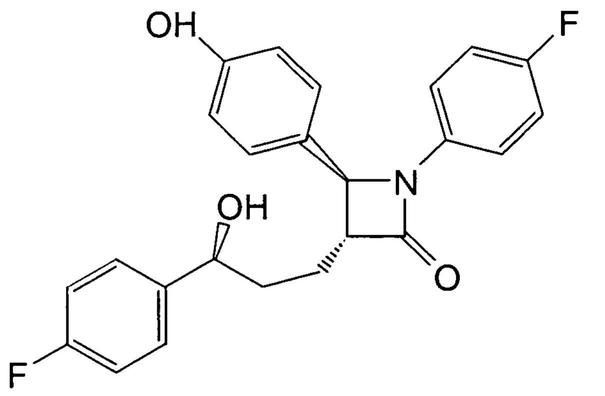

(II)

oder ein pharmazeutisch akzeptables Salz oder Solvat hiervon.

2. Therapeutische Kombination, umfassend:

(a) eine erste Menge von zumindest einem von Cholestyramin, Colestipol, Colesevelam hydrochlorid und Mischungen hiervon; und
(b) eine zweite Menge von zumindest einem Sterolabsorptionsinhibitor, repräsentiert durch die folgende Formel (II):

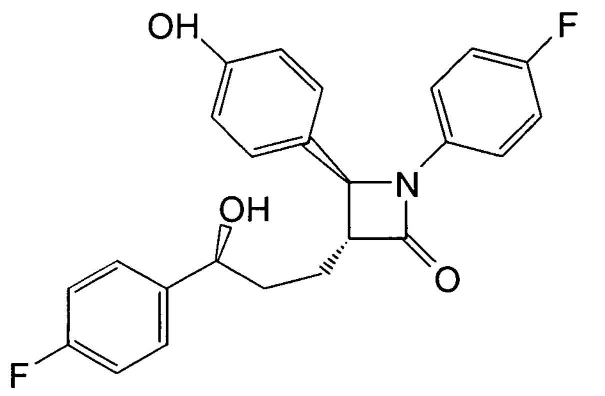

(II)

oder ein pharmazeutisch akzeptables Salz oder Solvat hiervon,

worin die erste Menge und die zweite Menge zusammen eine therapeutisch wirksame Menge zur Behandlung oder Vorbeugung von Diabetes, Fettleibigkeit oder eines vaskularen Zustands, der aus Hyperlipidämie, Atherosklerose und Hypercholesterinämie ausgewählt ist, umfassen.

3. Zusammensetzung oder therapeutische Kombination gemäß irgendeinem der Ansprüche 1 oder 2, worin das zumindest eine von Cholestyramin, Colestipol, Colesevelam hydrochlorid und Mischungen hiervon Cholestyramin umfaßt.

4. Zusammensetzung oder therapeutische Kombination gemäß Anspruch 3, worin das zumindest eine von Cholestyramin, Colestipol, Colesevelam hydrochlorid und Mischungen hiervon Colestipol umfaßt.

**5.** Zusammensetzung oder therapeutische Kombination gemäß irgendeinem der Ansprüche 1 oder 2, worin das zumindest eine von Cholestyramin, Colestipol, Colesevelam hydrochlorid und Mischungen hiervon an einen Säuger in einer Menge von 1 bis 50 g an Gallensäurekomplexbildner pro Tag zu verabreichen ist.

**6.** Zusammensetzung oder therapeutische Kombination gemäß irgendeinem der Ansprüche 1 oder 2, worin der zumindest eine Sterolabsorptionsinhibitor an einen Säuger in einer Menge von 0,1 bis 1.000 mg des Sterolabsorptionsinhibitors pro Tag zu verabreichen ist.

**7.** Zusammensetzung oder therapeutische Kombination gemäß irgendeinem der Ansprüche 1 oder 2, ferner umfassend zumindest eine Verbindung, die aus der Gruppe ausgewählt ist, die aus Lovastatin, Pravastatin, Fluvastatin, Simvastatin, Atorvastatin, Cerivastatin, CI-81, Pitavastatin, L-659,699, Squalestatin 1, NB-598 und DMP-565 besteht.

**8.** Zusammensetzung oder therapeutische Kombination gemäß Anspruch 7, worin die zumindest eine Verbindung aus der Gruppe ausgewählt ist, die aus Lovastatin, Pravastatin, Fluvastatin, Simvastatin, Atorvastatin, Cerivastatin und Mischungen hiervon besteht.

**9.** Zusammensetzung oder therapeutische Kombination gemäß Anspruch 8, worin die zumindest eine Verbindung Simvastatin ist.

**10.** Zusammensetzung oder therapeutische Kombination gemäß irgendeinem der Ansprüche 1 oder 2, ferner umfassend zumindest ein lipidsenkendes Mittel, das aus der Gruppe ausgewählt ist, die aus Clofibrat, Gemfibrozil, Ciprofibrat, Bezafibrat, Clinofibrat, Binifibrat, Lifibrol, Fenofibrat, Troglitazon, Rosiglitazon, Pioglitazon, Ciglitazon, Englitazon, Darglitazon, BRL 49653, C.A:S: Registrierungsnr. 317318-32-4, GW2331, Nikotinsäure, Niceritrol, Nicofuranose, Acipimox, Avasimibe, HL-004, Lecimibid, Probucol, AGI-1067, HOE-402, Flohsamen, Guar, Hafer, Pektin und Sitostanolester besteht.

**11.** Zusammensetzung oder therapeutische Kombination gemäß irgendeinem der Ansprüche 1 oder 2, ferner umfassend zumindest ein Additiv, das aus der Gruppe ausgewählt ist, die besteht aus Probucol, Tocopherol, Ascorbinsäure, β-Carotin, Selen, Vitamin $B_6$, Vitamin $B_{12}$, Adrogenen und Kombinationen hiervon, Östrogenen und Kombinationen hiervon, Progestinen, einer Kombination von Progestinen und Östrogenen, Diethylpropion, Mazindol, Phenylpropanolamin, Phentermin, Phendimetrazin, Phendamintartrat, Methamphetamin, Tartrat, Sibutramin, Fenfluroamin, Dexfenfluramin, Fluoxetin, Fluvoxamin, Paroxtin, Fefloxaton, Moclobemid, Brofaromin, Phenoxathin, Esupron, Befol, Toloxaton, Pirlindol, Amiflamin, Sercloremin, Bazinaprin, Lazabemid, Milacemid, Caroxazon, Orlistat, Argatroban, Bivalirudin, Dalteparinnatrium, Desirudin, Dicumarol, Lyapolatnatrium, Nafomastatmesylat, Phenprocoumon, Tinzaparinnatrium, Warfarinnatrium, Anagrelid hydrochlorid, Cilostazol, Danaparoidnatrium, Dazoxiben hydrochlorid, Efegatransulfat, Enoxaparinnatrium, Fluretofen, Ifetroban, Ifetrobannatrium, Lamifiban, Lotrafibanhydrochlorid, Napsagatran, Orbofibanacetat, Roxifibanacetat, Sibrafiban, Trifenagrel, Abciximab, Zolimom aritox, Tirofiban, Xemilofiban, monoklonaler Antikörper 7E3, Clopidogrelbisulfat, Epoprostenol, Epoprostenolnatrium, Ticlopidin hydrochlorid, Aspirin, Ibuprofen, Naproxen, Sulindae, Idomethacin, Mefenamat, Droxicam, Diclofenac, Sulfinpyrazon, Piroxicam, Dipyridamol, Acadesin, Beraprost, Beraprostnatrium, Ciprostencalcium, Itazigrel, Lifarizin, Oxagrelat, Fradafiban, Orbofiban, Naphthalin-2-sulfonsäure-{1-[3-aminoiminomethyl)benzyl]-2-oxo-pyrrolidin-3-(S)-yl} amidtrifluoracetat, Dibenzofuran-2-sulfonsäure-{1-[3-(aminomethyl)benzyl]-5-oxo-pyrrolidin-3-yl}amid, Toluol-4-sulfonsäure-{1-[3-(aminoiminomethyl)benzyl]-2-oxo-pyrrolidin-3-(S)-yl}amidtrifluoracetat, 3,4-Dihydro-1H-isochinolin-2-sulfonsäure-{1-[3-(aminoiminomethyl)-benzyl]-2-oxo-pyrrolidin-3-(S)-yl}amidtrifluoracetat, Clentiazemmaleat, Amlodipinbesylat, Isradipin, Nimodipin, Felodipin, Nilvadipin, Nifedipin, Teludipin hydrochlorid, Diltiazem hydrochlorid, Belfosdil, Verapamil hydrochlorid, Festodil, Fenspirid hydrochlorid, Labetalol hydrochlorid, Proroxan, Alfuzosin hydrochlorid, Acebutolol, Acebutolol hydrochlorid, Alprenolol hydrochlorid, Atenolol, Bunolol hydrochlorid, Carteolol hydrochlorid, Celiprolol hydrochlorid, Cetamolol hydrochlorid, Cicloprolol hydrochlorid, Dexpropranolol hydrochlorid, Diacetolol hydrochlorid, Dilevalol hydrochlorid, Esmolol hydrochlorid, Exaprolol hydrochlorid, Flestololsulfat, Labetalol hydrochlorid, Levobetaxolol hydrochlorid, Levobunolol hydrochlorid, Metalol hydrochlorid, Metoprolol, Metoprololtartrat, Nadolol, Pamatololsulfat, Penbutololsulfat, Practolol, Propranolol hydrochlorid, Sotalol hydrochlorid, Timolol, Timololmaleat, Tiprenolol hydrochlorid, Tolamolol, Bisoprolol, Bisoprololfumarat, Nebivolol, Benazepril hydrochlorid, Benazeprilat, Captopril, Delapril hydrochlorid, Fosinoprilnatrium, Libenzapril, Moexipril hydrochlorid, Pentopril, Perindopril, Quinapril hydrochlorid, Quinaprilat, Ramipril, Spirapril hydrochlorid, Spiraprilat, Teprotid, Enalaprilmaleat, Lisinopril, Zofenoprilcalcium, Perindoprilerbumin, Althiazid, Benzthiazid, Captopril, Carvedilol, Chlorothiazidnatrium, Clonidin hydrochlorid, Cyclothiazid, Delapril hydrochlorid, Dilevalol hydrochlorid, Doxazosinmexylat, Fosinoprilnatrium, Guanfacin hydrochlorid, Methyldopa, Metoprololsuccinat, Moexipril hydrochlorid, Monatepilmaleat, Pelanserin hydrochlorid, Phenoxybenzamin hydrochlorid, Prazosin hydrochlorid, Primidolol, Quinapril hydrochlorid, Quinaprilat, Ramipril, Terazosin hydrochlorid, Candesartan, Candesartancilexetil, Telmisartan, Amlodipinbesylat, Amlodipinmaleat, Bevantolol hydrochlorid, Candesartan, Irbesartan, Losartankalium, Candesartancilexetil, Telmisartan, Amlodipinbesylat, Amlodipinmaleat, Betaxolol hydrochlorid, Bevantolol hydrochlorid, Butoprozin hydrochlorid, Carvedilol, Cinepazetmaleat, Metoprololsuccinat, Molsidomin, Monatepilmaleat, Primidolol, Ranolazin hydrochlorid, Tosifen, Verapamil hydrochlorid, Fostedil, Azaclorzin hydrochlorid, Chromonar hydrochlorid, Clonitrat, Diltiazem hydrochlorid, Dipyridamol, Droprenilamin, Erythrityltetranitrat, Isosorbiddinitrat, Isosorbidmononitrat, Lidoflazin, Mioflazin hydrochlorid, Mixidin, Molsidomin, Nicorandil, Nifedipin, Nisoldipin, Nitroglycerin, Oxprenolol hydrochlorid, Pentrinitrol, Perhexilinmaleat, Prenylamin, Propatylnitrat, Terodilin hydrochlorid, Tolamolol, Verapamil, das Kombinationsprodukt von Hydrochlorothiazid und Spironolacton, das Kombinationsprodukt von Hydrochlorothiazid und Triamteren, Acetohexamid, Chlorpropamid, Gliamilid, Gliclazid, Glimepirid, Glipizid, Glyburid, Glibenclamid, Tolazamid, Tolbutamid, Repaglinid und Netaglinid, Metformin und Buformin, Acarbose, Miglitol, Carniglibose, Voglibose, Amlintid, Pramlintid und Exendin.

**12.** Pharmazeutische Zusammensetzung zur Behandlung oder Vorbeugung von Hyperlipidämie, Atherosklerose, Diabetes, Fettleibigkeit oder Hypercholesterinämie, umfassend eine therapeutisch wirksame Menge der Zusammensetzung von Anspruch 1 und einen pharmazeutisch akzeptablen Träger.

**13.** Verwendung einer Zusammensetzung gemäß irgendeinem der Ansprüche 1 bis 12 zur Herstellung eines Medikaments zur Vorbeugung oder Behandlung von Hyperlipidämie, Atherosklerose, Diabetes, Fettleibigkeit oder Hypercholesterinämie.

**14.** Verwendung gemäß Anspruch 13 zur Behandlung oder Vorbeugung von Fettleibigkeit.

**15.** Therapeutische Kombination gemäß Anspruch 2, worin das zumindest eine von Cholestyramin, Colestipol, Colesevelam hydrochlorid und Mischungen hiervon begleitend zu zumindest einem Sterolabsorptionsinhibitor, der durch die Formel (II) repräsentiert wird, verabreicht wird.

**16.** Therapeutische Kombination gemäß Anspruch 2, worin das zumindest eine von Cholestyramin, Colestipol, Colesevelam hydrochlorid und Mischungen hiervon und der Sterolabsorptionsinhibitor, der durch die Formel (II) repräsentiert wird, in separaten Behandlungszusammensetzungen vorliegen.

## Revendications

**1.** Composition comprenant :

a) au moins un agent choisi parmi les cholestyramine, colestipol et chlorhydrate de colesevelam et leurs mélanges,
b) et au moins un inhibiteur d'absorption de stérols, représenté par la formule (II) suivante :

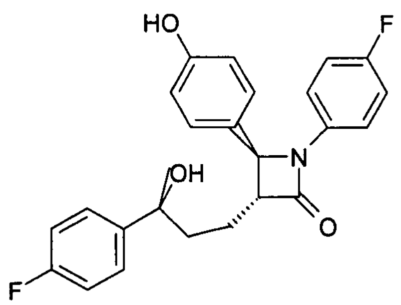

(II)

ou un sel ou solvat pharmacologiquement admissible de celui-ci.

**2.** Combinaison thérapeutique comprenant :

a) une première quantité d'au moins l'un des cholestyramine, colestipol et chlorhydrate de colesevelam et leurs mélanges,

b) et une deuxième quantité d'au moins un inhibiteur d'absorption de stérols, représenté par la formule (II) suivante :

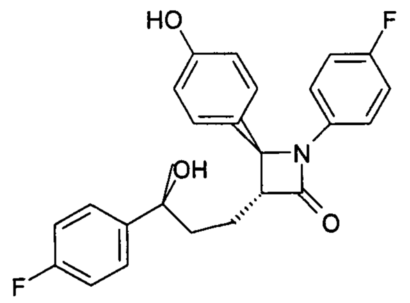

(II)

ou d'un sel ou solvat pharmacologiquement admissible de celui-ci,

dans laquelle la première quantité et la deuxième quantité constituent ensemble une quantité à effet thérapeutique pour le traitement ou la prévention du diabète, de l'obésité ou d'un état vasculaire choisi parmi une hyperlipidémie, une athérosclérose et une hypercholestérolémie.

**3.** Composition ou combinaison thérapeutique conforme à la revendication 1 ou 2, dans laquelle ledit agent choisi parmi les cholestyramine, colestipol et chlorhydrate de colesevelam et leurs mélanges, au nombre d'au moins un, comprend de la cholestyramine.

**4.** Composition ou combinaison thérapeutique conforme à la revendication 3, dans laquelle ledit agent choisi parmi les cholestyramine, colestipol et chlorhydrate de colesevelam et leurs mélanges, au nombre d'au moins un, comprend du colestipol.

**5.** Composition ou combinaison thérapeutique conforme à la revendication 1 ou 2; avec laquelle ledit agent choisi parmi les cholestyramine, colestipol et chlorhydrate de colesevelam et leurs mélanges, au nombre d'au moins un, est destiné à être administré à un mammifère en une quantité de 1 à 50 grammes de chélateur d'acides biliaires par jour.

**6.** Composition ou combinaison thérapeutique conforme à la revendication 1 ou 2, avec laquelle ledit inhibiteur d'absorption de stérols, au nombre d'au moins un, est destiné à être administré à un mammifère en une quantité de 0,1 à 1000 milligrammes d'inhibiteur d'absorption de stérols par jour.

**7.** Composition ou combinaison thérapeutique conforme à la revendication 1 ou 2, qui comprend en outre au moins un composé choisi dans l'ensemble constitué par les lovastatine, pravastatine, fluvastatine, simvastatine, atorvastatine, cérivastatine, CI-981, pitavastatine, L-659.699, squale-statine 1, NB-598 et DMP-565.

**8.** Composition ou combinaison thérapeutique conforme à la revendication 7, dans laquelle ledit composé au nombre d'au moins un est choisi parmi les lovastatine, pravastatine, fluvastatine, simvastatine, atorvastatine et cérivastatine, ainsi que leurs mélanges.

**9.** Composition ou combinaison thérapeutique conforme à la revendication 8, dans laquelle ledit composé au nombre d'au moins un est de la simvastatine.

**10.** Composition ou combinaison thérapeutique conforme à la revendication 1 ou 2, qui comprend en outre au moins un agent abaissant les taux de lipides, choisi dans l'ensemble formé par les clofibrate, gemfibrozil, ciprofibrate, bézafibrate, clinofibrate, binifibrate, lifibrol, fénofibrate, troglitazone, rosiglitazone, pioglitazone, ciglitazone, englitazone, darglitazone, BRL 49653, N° CAS 317318-32-4, GW2331, acide nicotinique, nicéritrol, nicofuranose, acipimox, avasimibe, HL-004, lécimibide, probucol, AGI-1067, HOE-402, psyllium, guar, avoine, pectine et ester de sitostanol.

**11.** Composition ou combinaison thérapeutique conforme à la revendication 1 ou 2, qui comprend en outre au moins un adjuvant choisi dans l'ensemble formé par les probucol, tocophérol, acide ascorbique, β-carotène, sélénium, vitamine $B_6$, vitamine $B_{12}$, androgènes et leurs combinaisons, oestrogènes et leurs combinaisons, progestines, combinaisons de progestines et d'oestrogènes, diéthylpropion, mazindol, phénylpropanolamine, phentermine, phendimétrazine, tartrate de phendamine, tartrate de méthamphétamine, sibutramine, fenfluramine, dexfenfluramine, fluoxétine, fluvoxamine, paroxétine, befloxatone, moclobémide, brofaromine, phénoxathine, ésuprone, befol, toloxatone, pirlindol, amiflamine, serclorémine, bazinaprine, lazabémide, milacémide, caroxazone, orlistat, argatroban, bivalirudine, daltéparine sodique, désirudine, dicumarol, lyapolate sodique, mésylate de nafomastat, phénprocoumone, tinzaparine sodique, warfarine sodique, chlorhydrate d'anagrélide, cilostazol, danaparoïde sodique, chlorhydrate de dazoxibène, sulfate d'éfégatran, énoxaparine sodique, flurétofène, ifétroban, ifétroban sodique, lamifiban, chlorhydrate de lotrafiban, napsagatran, acétate d'orbofiban, acétate de roxifiban, sibrafiban, trifénagrel, abciximab, zolimomab aritox, tirofiban, xémilofiban, anticorps monoclonal 7E3, bisulfate de clopidogrel, époprosténol, époprosténol sodique, chlorhydrate de ticlopidine, aspirine, ibuprofène, naproxène, sulindac, indométhacine, méfénamate, droxicam, diclofénac, sulfinpyrazone, piroxicam, dipyridamole, acadésine, béraprost, béraprost sodique, ciprostène calcique, itazigrel, lifarizine, oxagrélate, fradafiban, orbofiban, trifluoroacétate de {1-[3-(amino-imino-méthyl)benzyl]-2-oxo-pyrrolidin-3-(S)-yl}amide d'acide naphtalène-2-sulfonique, {1-[3-(amino-méthyl)benzyl]-5-oxo-pyrrolidin-3-yl} amide d'acide dibenzofuranne-2-sulfonique, trifluoroacétate de {1-[3-(amino-imino-méthyl)benzyl]-2-oxo-pyrrolidin-3-(S)-yl}amide d'acide toluène-4-sulfonique, trifluoroacétate de {1-[3-(amino-imino-méthyl)benzyl]-2-oxo-pyrrolidin-3-(S)-yl}amide d'acide dihydro-1H-isoquinoléine-2-sulfonique, maléate de clentiazem, bésylate d'amlodipine, isradipine, nimodipine, félodipine, nilvadipine, nifédipine, chlorhydrate de téludipine, chlorhydrate de diltiazem, belfosdil, chlorhydrate de vérapamil, fostédil, chlorhydrate de fenspiride, chlorhydrate de labétalol, proroxan, chlorhydrate d'alfuzosine, acébutolol, chlorhydrate d'acébutolol, chlorhydrate d'alprénolol, aténolol, chlorhydrate de bunolol, chlorhydrate de cartéolol, chlorhydrate de céliprolol, chlorhydrate de cétamolol, chlorhydrate de cicloprolol, chlorhydrate de dexpropranolol, chlorhydrate de diacétolol, chlorhydrate de dilévalol, chlorhydrate d'esmolol, chlorhydrate d'exaprolol, sulfate de flestolol, chlorhydrate de labétalol, chlorhydrate de lévobétaxolol, chlorhydrate de lévobunolol, chlorhydrate de métalol, métoprolol, tartrate de métoprolol, nadolol, sulfate de pamatolol, sulfate de penbutolol, practolol, chlorhydrate de propranolol, chlorhydrate de sotalol, timolol, maléate de timolol, chlorhydrate de tiprénolol, tolamolol, bisoprolol, fumarate de bisoprolol, nébivolol, chlorhydrate de bénazépril, bénazéprilate, captopril, chlorhydrate de délapril, fosinopril sodique, libenzapril, chlorhydrate de moexipril, pentopril, perindopril, chlorhydrate de quinapril, quinaprilate, ramipril, chlorhydrate de spirapril, spiraprilate, téprotide, maléate d'énalapril, lisinopril, zofénopril calcique, perindopril erbumine, althiazide, benzthiazide, captopril, carvédilol, chlorothiazide sodique, chlorhydrate de clonidine, cyclothiazide, chlorhydrate de délapril, chlorhydrate de dilévalol, mésylate de doxazosine, fosinopril sodique, chlorhydrate de guanfacine, méthyl-dopa, succinate de métoprolol, chlorhydrate de moexipril, maléate de monatépil, chlorhydrate de pélansérine, chlorhydrate de phénoxybenzamine, chlorhydrate de prazosine, primidolol, chlorhydrate de quinapril, quinaprilate, ramipril, chlorhydrate de térazosine, candésartan, candésartan cilexétil, telmisartan, bésylate d'amlodipine, maléate d'amlodipine, chlorhydrate de bévantolol, candésartan, irbésartan, losartan potassique, candésartan cilexétil, telmisartan, bésylate d'amlodipine, maléate d'amlodipine, chlorhydrate de bétaxolol, chlorhydrate de bévantolol, chlorhydrate de butoprozine, carvédilol, maléate de cinépazet, succinate de métoprolol, molsidomine, maléate de monatépil, primidolol, chlorhydrate de ranolazine, tosifène, chlorhydrate de vérapamil, fostédil, chlorhydrate d'azaclorzine, chlorhydrate de chromonar, clonitrate, chlorhydrate de diltiazem, dipyridamole, droprénilamine, tétranitrate d'érythrityle, dinitrate d'isosorbide, mononitrate d'isosorbide, lidoflazine, chlorhydrate de mioflazine, mixidine, molsidomine, nicorandil, nifédipine, nisoldipine, nitroglycérine, chlorhydrate d'oxprénolol, pentrinitrol, maléate de perhexiline, prénylamine, nitrate de propatyle, chlorhydrate de térodiline, tolamolol, vérapamil, produit de combinaison d'hydrochlorothiazide et de spironolactone, produit de combinaison d'hydrochlorothiazide et de triamtérène, acétohexamide, chlorpropamide, gliamilide, gliclazide, glimépiride, glipizide, glyburide, glibenclamide, tolazamide, tolbutamide, répaglinide, natéglinide, metformine, buformine, acarbose, miglitol, camiglibose, voglibose, amlintide, pramlintide et exendine.

**12.** Composition pharmaceutique, conçue pour le traitement ou la prévention d'une hyperlipidémie, de l'athérosclérose, d'un diabète, de l'obésité ou de l'hypercholestérolémie, comprenant une composition conforme à la revendication 1, en une quantité à effet thérapeutique, et un véhicule pharmacologiquement admissible.

**13.** Emploi d'une composition conforme à l'une des revendications 1 à 12, en vue de la fabrication d'un médicament conçu pour le traitement ou la prévention d'une hyperlipidémie, de l'athérosclérose, d'un diabète, de l'obésité ou de l'hypercholestérolémie,

**14.** Emploi, conforme à la revendication 13, en vue du traitement ou de la prévention d'une hyperlipidémie.

**15.** Combinaison thérapeutique conforme à la revendication 2, dans laquelle ledit agent choisi parmi les cholestyramine, colestipol et chlorhydrate de colesevelam et leurs mélanges, au nombre d'au moins un, est administré conjointement avec ledit inhibiteur d'absorption de stérols au nombre d'au moins un, représenté par la formule (II).

**16.** Combinaison thérapeutique conforme à la revendication 2, dans laquelle ledit agent choisi parmi les cholestyramine, colestipol et chlorhydrate de colesevelam et leurs mélanges, au nombre d'au moins un, et ledit inhibiteur d'absorption de stérols représenté par la formule (II) se trouvent dans des compositions de traitement distinctes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5767115 A
- US 5624920 A
- US 5668990 A
- US 5656624 A
- US 5688787 A
- US 5846966 A
- US 5661145 A
- WO 0038725 A
- US 5698527 A
- WO 9535277 A
- WO 02081454 A
- WO 0250068 A
- WO 0250060 A
- WO 0250027 A
- US 5631365 A
- US 6207822 B
- US 27928801 P
- WO 9302048 A
- WO 9728149 A
- US 3948973 A
- US 3781328 A
- US 3716583 A
- BE 884722
- US 6028109 A
- WO 0075103 A
- WO 9843081 A
- WO 9805331 A
- WO 0076488 A
- US 5994554 A
- US 5859051 A
- WO 9920275 A
- WO 9938845 A
- WO 0063161 A
- WO 0100579 A
- WO 0112612 A
- WO 0112187 A
- WO 9731907 A
- WO 0100603 A
- US 5093365 A
- WO 9904815 A
- US 6248781 B
- WO 0023416 A
- WO 0023415 A
- WO 0023425 A
- WO 0023445 A
- WO 0023451 A
- WO 0063153 A
- WO 9725042 A
- WO 0063190 A
- WO 0121181 A
- WO 0116120 A
- WO 0063196 A
- WO 0063209 A
- US 6008237 A
- WO 0078312 A
- WO 0078313G A
- US 6166049 A
- WO 0117994 A
- WO 0125225 A
- WO 0125226 A
- WO 0114349 A
- WO 0114350 A
- WO 0104351 A
- WO 0050392 A
- WO 0053563 A
- WO 9946232 A
- WO 9912534 A
- WO 9915520 A
- WO 0121578 A
- WO 0140192 A
- WO 0038727 A
- WO 0038721 A
- US 6147090 A
- US 6121319 A
- US 6147250 A
- WO 9938498 A

### Non-patent literature cited in the description

- **THOMPSON G.R. et al.** *Expert opinion on investigational drugs,* December 2000, vol. 9 (11
- **M. HUETTINGER et al.** Hypolipidemic activity of HOE-402 is Mediated by Stimulation of the LDL Receptor Pathway. *Arterioscler. Thromb.,* 1993, vol. 13, 1005-12
- **H. GYLLING et al.** Serum Sterols During Stanol Ester Feeding in a Mildly Hypercholesterolemic Population. *J. Lipid Res.,* 1999, vol. 40, 593-600